# EUROPEAN PATENT SPECIFICATION

(11) **EP 1 971 685 B1**
(45) Date of publication and mention of the grant of the patent: **19.08.2009**
(21) Application number: 07700476.0
(22) Date of filing: 11.01.2007
(51) Int. Cl.: C12N 15/10, C12N 15/62, C40B 40/10, C40B 40/08

(54) **A METHOD FOR DEVELOPING A TISSUE PROTEOME LIBRARY**
VERFAHREN ZUR ENTWICKLUNG EINER GEWEBE-PROTEOMBIBLIOTHEK
PROCEDE DE DEVELOPPEMENT D'UNE BIBLIOTHEQUE PROTEOMIQUE TISSULAIRE

(30) Priority: 12.01.2006 IN DE00962006
(43) Date of publication of application: 24.09.2008
(73) Proprietor: Council of Scientific and Industrial Research, New Delhi 110 001 (IN)
(72) Inventor: MURTHY, Satyanarayana, Bulusu, Hyderabad 500 007, Andhrapradesh (IN)
(74) Representative: Smithson, Robert Alan
(86) International application number: PCT/IB2007/000078
(87) International publication number: WO 2007/080504

(56) References cited:
- GILLETTE WILLIAM K ET AL: "Pooled ORF expression technology (POET) - Using proteomics to screen pools of open reading frames for protein expression" MOLECULAR & CELLULAR PROTEOMICS, vol. 4, no. 11, November 2005 (2005-11), pages 1647-1652, XP002431137 ISSN: 1535-9476
- STUDIER F W ET AL: "USE OF T7 RNA POLYMERASE TO DIRECT EXPRESSION OF CLONED GENES" METHODS IN ENZYMOLOGY, ACADEMIC PRESS INC, SAN DIEGO, CA, US, vol. 185, 1990, pages 60-89, XP000647676 ISSN: 0076-6879
- HOLZ CATERINA ET AL: "A human cDNA expression library in yeast enriched for open reading frames" GENOME RESEARCH, vol. 11, no. 10, October 2001 (2001-10), pages 1730-1735, XP002431286 ISSN: 1088-9051

## Description

### Field of the invention:

The present invention relates to a method for developing a tissue proteome library, useful in making a representative library of the "truly" expressed proteins and overexpressing a large number of transcripts (mRNAs) present in a given tissue.

### Background of the invention:

Cells derived from any part of a plant could regenerate the complete plant, while vertebrate cells are devoid of this ability. Newly formed zygote alone possesses such an ability to develop into a complete organism. Researchers attempting to clone mammals achieved very low success rates, as mammalian cloning by transfer of growth arrested adult somatic cell nucleus into mature oocytes is still in its developing phase and it is not yet clear what technical and biological factors underlie and limit this process (Solter, 2000). Reproduction in vertebrates involves complex sets of differentiation events that lead to development of a fully-grown animal. Recent success in mammalian cloning revealed that a growth-arrested normal somatic cell nucleus is capable of developing into a fully-grown animal upon transplantation into an enucleated mature oocyte (Campbell et al., 1996 and Wilmut et al., 1997). These studies suggested that the factors necessary and sufficient for bringing about de-differentiation of differentiated nucleus and its reprogramming are present in the ooplasm of mature oocytes and opened newer avenues for studying complex processes like nuclear-reprogramming, imprinting, embryonic differentiation and development (Coleman, 2002 and Tsunoda and Kato, 2002). Possible applications of this technology include reproductive cloning of farm animals and therapeutic cloning of humans for autologous stem cell transplants (Campbell et al., 2001 and Westhusin et al., 2001). For achieving higher rates of success in mammalian cloning, nuclear reprogramming, embryonic differentiation and development must be understood at their molecular levels. Proteins and mRNAs of maternal origin present in oocytes have been proposed to assist in zygotic reprogramming, differentiation and development, while additional factors required for these processes must be transcribed from the zygotic nucleus (Lieb et al., 1998, Paynton, 1998 and Ryabova, 1994). For practical, ethical and pragmatic reasons only milligram quantities of mammalian oocyte and early embryonic tissues could be procured, which are not sufficient for isolation, identification and characterization of potentially interesting proteins present in such tissues.

One of the major, present day health disorders of mankind is carcinoma of various human tissues that result in uncontrolled cell division. Researchers have been striving hard to understand, classify and define tumor states in terms of molecules they contain (Wulfkuhle et al., 2003), employing DNA microarray and proteomics approaches. These studies have been aimed at identifying biomarkers useful in diagnosis, prognosis and therapeutic application in tumor treatments (Amatschek et al., 2004 and Vaibhav et al., 2005). Availability of tumor tissue specimens from cancer patients shall be very limited and hardly would they be sufficient for isolation / identification of interesting, particularly low abundant proteins contributing to these disorders. In view of the above, cDNA libraries are constructed from available tissues and transcripts present in such libraries analyzed employing the microarray approach. Microarray quantifies levels of transcripts present in cDNA libraries of target and relevant control tissues for arriving at crucial genes expressed in the target tissues. As proteins are the functional molecules involved in most of the biological processes, it is perhaps more advantageous to isolate and conduct structure-function study of proteins involved in physiological functions directly.

Methods have been reported in the literature for construction of cDNA libraries from tissues (Ravassard et al., 1997) and analysis of transcripts present in such libraries by microarray (Giltnane and Rimm, 2004). These analyses determine only the relative abundance of transcripts in terms of their genres arrayed on microarray slides as compared to those of appropriate control tissue cDNA libraries. This implies that microarray does not reveal identity and abundance of individual and particularly unknown transcripts (mRNAs) of genes other than those arrayed on the microarray slide. Although genes are the functional units of genetic material, individual proteins expressed through their mRNAs from the genes actually contribute to the cellular processes. In view of this, studying the proteins translated from their mRNAs present in tissues directly might be more advantageous for understanding their cellular functions. Functional molecules of biological systems being proteins translated from mRNAs present in cells, structure-function study of proteins is obligatory for understanding pathways involved in and regulation of cellular processes. Proteins could be isolated by conventional purification techniques from tissues, where availability of specific protein in the tissue on one side and tissues themselves on the other are not limited. Very frequently identification of all the proteins present in a tissue itself is extremely difficult due to lower abundance of many important proteins, even when the tissues are not limited in availability. Some of the important tissues like oocytes, early embryos, carcinoma / tissue specimen samples from patients etc. are available only in very limited amounts. Therefore, interesting transcripts present in such tissues must be amplified, cloned, proteins expressed, identified and purified before any study is possible about their structure and function. If technologies are developed that can overexpress all the transcripts present in such tissues, it would at least enable identification of important transcripts expressed in such tissues.

Present invention directly relates to an article entitled "Characterization of novel DNA-binding proteins expressed in snake oocyte cDNA library", appeared in Protein Expression and Purification, Volume: 53, 2007, pages 164-178, by Mala, G., Paithankar, K. R., Jagannadham, M. V., Sundaram, C. S., Murthy, B. S.* and Singh, L., while it poseeses some background relevance to another article entitled "Pooled ORF expression technology (POET) - Using proteomics to screen pools of open reading frames for protein expression". MOLECULAR & CELLULAR PROTEOMICS, vol: 4, no 11, November 2005 (2005-11), pages 1647 - 1652, XP002431137 ISSN: 1535-9476, by GILETTE WILLIAM K et al.

Present post-genomic era provides greater opportunities for proteome-wide investigations. Most recent innovations in functional assessment of genes include microarray and proteomics technologies. Proteomics being a powerful technique possesses all the potential for providing such an opportunity. Proteomics, recently endowed with automated high throughput capabilities is able to analyze / identify even large number of proteins, when present even at extremely low levels. Proteomics employs two dimensional polyacrylamide gel electrophoresis (2D PAGE) for resolving protein pools, in-gel enzyme digestion of individual proteins for generating peptide mixture and Matrix Assisted Laser Desorption / Ionization Time of Flight (MALDI-TOF) for generating Peptide Mass Fingerprint (PMF) data of the protein. PMF data is stored and communicated as protein databases, which provides information on protein expression profiles (Hochstrasser et al., 2002) of the tissues. 2D PAGE is the only tested method available for quantitatively comparing changes in protein profiles of cells, tissues or whole organisms (Nordhoff et al., 2001 and Mann et al., 2001).

In view of the above, there is need for developing technologies for construction of cDNA expression libraries that overexpress all the transcripts present in tissues, which can be used in identifying important factors expressed in tissues under specialized physiological conditions. PCR amplification of mRNA pool after reverse-transcription to cDNA from tissues available in limited amounts shall facilitate construction of total cDNA library and expression of entire library for isolation and identification of important and low abundant transcripts preset in the library. Bacteria (*E. coil*), being a simple microorganism, are extensively used for overexpression of variety of proteins (Panda, 2003). Bacteria constitute autonomously dividing cells that express genes cloned in them as plasmids, in the presence of suitable inducers. Thus we expected that bacteria should overexpress proteins even when a pool of cDNAs are cloned in them and help in overproducing proteins from all the cloned transcripts of the library. Since high throughput proteomic methods have become available, it would be possible to conduct studies on large number of proteins overexpressed in such libraries. Isolation and identification of all the proteins made in cells and tissues enable proteome-wide investigations, which could enhance our understanding of the mechanisms involved in fundamental processes of life. Present approach of constructing tissue proteome library by cloning and expressing entire library of cDNAs, combined with proteomics analysis for identification of novel factors expressed in the library demonstrates potential application of these methods in proteome investigations, in particular where tissue availability is a serious limitation in protein isolation / identification.

### Theoretical basis for developing a tissue proteome library by cloning cDNA pool of the tissue in all the three frames of an expression vector:

If we clone a pool of cDNAs of any tissue in an expression vector (frame a, b or c) one third of the cloned transcripts shall automatically be in the right frame for expression as there are only three frames of expression. By cloning the same cDNA pool in the other two frames, remaining two thirds of the clones can also be brought into correct frames for expression. By combining the plasmid pools cloned in frames a, b and c of an expression vector, we get all the transcripts of a tissue into the library in correct frames for expression of all the proteins. Upon induction, such a library should express (see later) all the tissue cDNAs to give rise to total cellular proteins, because of which the library is designated as a "Tissue Proteome Library". Questions arise as to what happens to the cDNAs not cloned in right reading (incorrect) frames for expression.

There are only 64 triplet codons, of which three are termination codons. This works out to one termination codon for every twenty-one normal codons, which shall be the frequency of occurrence of these codons in incorrect frame cloned cDNAs for expression. This is because biological systems make sure that termination codons do not occur in the coding regions of the transcripts. Considering average mass of an amino acid as 115 Da, transcripts cloned in incorrect frames for expression should get truncated as peptides of about 21 amino acids, which will have a mass of (21x115=) ∼2.42 kDa. Even if occurrence of stop codon frequency lowers by 300%, such truncated peptides shall possess only a mass of ∼9.66 kDa. Thus all the proteins of mass values >10 kDa expressed in tissue proteome libraries (constructed as explained above by cloning cDNA pool of a tissue in all three frames of an expression vector) should theoretically be physiological proteins, while incorrect frame expressed proteins shall get truncated to generate peptides of <10 kDa. Proteomics usually employs 10-15% two-dimensional polyacrylamide gels for resolving proteins of mass range 10-100 kDa, which only can be resolved in these gels. Therefore, all the proteins overexpressed in tissue proteome libraries and resolved in such gels shall naturally be physiological proteins.

Bacteria constitute autonomously dividing cells that express transcripts cloned in them as plasmids, in the presence of suitable inducers. Thus we expected that bacteria should express proteins even when a pool of cDNAs are cloned in them and help in overproducing a total library of the cloned transcripts, particularly because we shall be inducing the library only for a few (2-3) hours. Since bacteria express proteins of a big range in size and as they possess no ability to discriminate between their-own and cloned transcripts in plasmid vectors, they should express the proteins, irrespective of the size of the cDNAs cloned in them. In view of the above, construction and expression of tissue proteome libraries forms an attractive method and potential strategy for expression of all the cDNAs of even unknown proteins of a tissue just in one go and facilitate in studying all the expressed proteins employing proteomics approach. In addition, proteomics possesses the ability to analyze and identify proteins, irrespective of whether analyzed proteins are of full-length or truncated. Therefore, this technology shall be useful in identifying novel proteins, even if truncated products of cDNAs are cloned and expressed (see protocols) or partially degraded proteins of 10-100 kDa are produced during protein purification from bacteria.

### Summary of the invention:

Construction of cDNA library enables profiling all the transcripts (mRNAs) present in tissues and their analysis by techniques like microarray. But each transcript encodes a specific protein, which can be identified and studied, only after cloning each transcript in correct frame in an expression vector, expressed in suitable hosts like bacteria / yeast and protein purified. First step towards this Herculean task is to identify all / novel transcripts present in a tissue, for which clear cut and straight forward methods are not available as yet. As there are three frames for cloning and one correct frame for expression, cloning a pool of cDNAs leads to uncertainty about what frame each transcript is cloned, which clones would be expressed and raises concerns about transcripts expressed in incorrect reading frames, leading to translation of non-physiological / hypothetical proteins. Procedures capable of automatically cloning all the transcripts of a tissue in correct frames for expression are also not available. However, it would be very advantageous to clone and express all the cDNA transcripts of a tissue in correct frames and purify expressed proteins to enable their identification upon resolving them by electrophoretic or chromatographic techniques. Such methodologies shall also help in identifying all, including low abundant transcripts present in scarce tissues like clinical specimens from patients, oocytes and early embryos. We developed a novel approach for cloning and explained the rationale for successful expression of all the transcripts present in a tissue in all three frames of an expression vector to develop a tissue proteome library.

Accordingly the present invention relates to description of a novel method for developing tissue proteome library, which overexpresses all the transcripts (mRNAs) present in a given tissue. Transcripts of interest present in a tissue are normally cloned and overexpressed individually to enable purification of expressed protein and for conducting its structure-function studies. Methods for identification of novel and low abundant transcripts present in tissues are not available, particularly of specimen tissue samples, oocytes and early embryos, for which tissue availability is a serious limitation. Expression of all the transcripts present in a tissue and comparison of the profile of total expressed protein with that of appropriate controls can be used in identification of all and particularly novel transcripts present in a tissue. This novel tissue proteome library construction approach enables expression of all the transcripts present in a tissue just in one go and analysis of all the expressed proteins employing proteomics and / or other suitable approaches.

### Brief description of figures and Tables:

- **Fig. 1**: shows the spread of expressed protein in the range of pI and mass.
- **Fig. 2**: shows Profile of purified DNA-binding proteins.
- **Fig. 3**: shows MALDI TOF spectra of four identified (SSP. Nos: 1405, 1955, 6601 & 7303) DNA- binding proteins.
- **Fig. 4**: shows ESI MS spectra of four of the studied proteins.

- **Table 1:**: Data of 25 cDNAs (from NCBI), analyzed employing the translation and pI / Mol. Wt. tools available at ExPASy proteomics server. Data includes stop codons present in the cDNAs and protein / peptide masses arrived at by these analyses.
- **Table 2:**: Data of the DNA-Binding Proteins Identified in the study by the Proteomics Analysis:
- **Table 3:**: Snake oocyte DNA-binding proteins identified with available sequence motifs and their predicted physiological function.
- **Table 4:**: Comparison of the protein identities obtained by PMF analysis with those identified by NCBI BLAST, employing their ESI MS sequence tags

### Detailed description:

From the above discussions we propose that, upon cloning a pool of cDNAs present in a tissue in all three frames (a, b and c) of an expression vector and pooling the clones one can obtain a tissue proteome library. Upon expression of such a library, one will find expression of at least most of the proteins present in the tissue and all expressed proteins shall be physiological proteins.
**1. Tissue proteome library construction from tissues:** A tissue proteome library can be constructed starting from a tissue, as described herein. One can synthesize total cDNA starting from as little as 10-100 ng of total or 2-50 ng of mRNA pool isolated from a tissue employing 5' and 3' specific primers. The cDNA pool obtained can be PCR amplified with 5' and 3' primers consisting of different and specific 6-(or more)-base-specific restriction enzymes sites (possessing sequences of which are known to occur only rarely in the genes) incorporated on the 5' and 3' termini (similar to those provided in the library construction kits available from popular Biotech companies). The 5' and 3' termini of the cDNAs can be restriction digested with the respective restriction enzymes and cDNA pool obtained can be cloned in all the three frames of expression vector, a, b and c. Choice of the vectors is described in section 3, below. Plasmids obtained thus can be transformed into suitable bacterial hosts that express cloned transcripts. Incidentally, earlier investigators at CCMB had developed a salt inducible expression system (host GJ1158, Patent No: 5830690, 3^{rd} November, 1998), which expresses most of the cloned transcripts in their native soluble form (Bhandari and Gowrishankar, 1997). This host requires only 300 mM sodium chloride (which is inexpensive) as an inducer for overexpression of the cloned genes. Further, GJ 1158 overexpresses cloned transcripts into proteins which are usually soluble. Plasmid pools obtained above could thus be transformed into electro-competent GJ 1158 bacteria by electroporation and clones obtained were grown on Luria Broth without sodium chloride (LBON)-agar plates. For obtaining better representation of the clones in the libraries, several transformations should be performed for each of the plasmid pools cloned in a, b and c and such pools combined independently, for each of the frame of expression. Libraries should be prepared both in GJ1158 as well as DH5α bacteria with plasmid pools a, b and c and stored as glycerol stocks at -80° C, as plasmids are unstable in expression hosts. Presence of 6xHis-tag coding sequence on the 5' terminus of cloned cDNAs in the vectors helps in generating 6xHis amino acid-tag on the N-termini of the proteins, which is useful in purification of library expressed proteins employing NiNTA-agarose chromatography.
**2. Procedure for constructing a tissue proteome library from existing tissue cDNA libraries:**
   Alternatively one can construct a cDNA library even from existing cDNA libraries. One can take a well-represented cDNA library, release all the cDNAs employing suitable restriction enzymes and ligate them into all three frames (a, b and c) of an expression vector, to get a tissue proteome library. Vectors that express cloned cDNAs in all three reading frames should be used and cDNAs should be cloned in all three frames and the libraries combined, for obtaining a tissue proteome library. Choice of vectors is described in section 3, below. For this purpose we must select expression vector that is available in forms (a, b and c) that can express cloned transcripts in all the three frames. In a cDNA library different restriction sites shall be present both on the 5' and 3' termini of the cloned cDNAs. We also must look for two different 6(or more)-base-specific restriction sites, which are present one each on the 5' and 3' termini of the cloned cDNAs and the same sites must also be present in the expression vector (in which the cDNAs are proposed to be cloned) in the 5' → 3' order. If we digest the library with two of such restriction enzymes, we shall release all the cDNAs with different and specific 5' and 3' termini. The cDNAs released from the library can then be cloned in all three forms (a, b and c) of the considered vector, and the libraries combined for getting a tissue proteome library.
   Since 6(or more)-base restriction enzyme sites are employed (assuming usage of only 6-base restriction enzymes), same 6-base sequence shall occur only once in. 4⁶ nucleotides (4x4x4x4x4x4 = 4096) within the transcripts. Large number of proteins present in a tissue shall be between 10-100 kDa, which can be resolved well only on 10-15%, 2D PAGE. Average mass of an amino acid being 115 Da, these proteins shall possess a maximum of 870 amino acids. Thus their cDNAs in general shall not be larger than 2.61 kb. Frequency of occurrence of 6-base restriction enzyme sites in cDNAs being only once in 4096 nucleotides, chances of internal truncation of cDNAs shall be extremely low. Therefore one can obtain, "two 6-(or more) base restriction enzyme-digested" cDNA-pool from a well-represented tissue cDNA library and clone the transcripts into the same restriction sites in all the frames of expression vectors for developing a tissue proteome library.
**3a. Choice of vectors for cloning cDNAs released from existing cDNA libraries and choice of alternative method of PCR amplification of cDNAs, while starting from a tissue:**
   For cloning the cDNAs obtained in the above procedures 1 and 2, one can use any set of expression vectors (a) containing a fusion protein tag at the 5' termini of the multiple cloning sites (MCS) and (b) available in three forms that express cloned cDNAs in all three reading frames, upon library induction. Several commercial vectors are available in the biotech market. An example for this is pET 28. But cloning the cDNAs in all three frames of pET 28 is neither necessary nor limited to this vector for constructing a tissue proteome library.
   PCR amplification of the cDNAs obtained from tissues can be performed in two distinct ways. In general in PCR amplification protocol, 5' and 3' specific primers can be employed and cDNAs recovered can be cloned in three expression vectors that express cloned transcripts in all three frames of expression. Alternatively, three 5'-specific primers that offset cloned cDNA transcripts by one, two and three nucleotides at the 5' termini can be employed such that the combined cDNA pool, obtained from all the PCR reactions can be cloned in any one expression vector possessing fusion protein tag at the 5' termini and the cloned transcripts can automatically be expressed in all the three reading frames.
**3b. Few clarifications and answers to questions likely to arise from Tissue Proteome Library construction procedures described above:**
   In most of the libraries constructed employing the available procedures; cDNAs are complete with respect to 3' termini, while they are largely incomplete with respect to 5' termini. Thus, by employing these procedures, we shall be cloning only the coding regions of the cDNAs into MCS of the vectors, because of which 5' un-translated regions of cDNAs are usually not cloned. Thus 5' un-translated regions of cDNAs neither get cloned nor get expressed, causing any un-certainty in the frame of protein expression. Further, N-terminal truncated proteins produced upon expression of such cDNAs can still be analyzed successfully by proteomics for protein identification.
   In a few methods that claim synthesis of complete 5' termini of cDNAs, 5' un-translated regions of cDNAs however do not get amplified beyond 10-20 nucleotides upstream of the initiator ATG codon. Inspection of the 5' un-translated regions of several dozens of cDNAs revealed that up to 30 nucleotides, these regions usually do not carry stop codons. Thus even if 10-20 nucleotides of 5' un-translated regions are synthesized, cloned into MCS of vectors and expressed; such expressed proteins neither suffer due to truncation on account of stop codons nor hinder proteomics based protein identification. The cDNAs cloned and expressed in the vectors usually produce peptides of about 30 amino acids, including the fusion peptide tags (His-tag / GST-tag /Thrombin), which also do not cause any problem in protein identification by proteomics. Although expected mass of peptides in wrong-frame expressed and truncated proteins is only about 2.3 kDa (see theoretical basis for developing a tissue proteome library, presented earlier), we have fixed a value of 10 kDa, to eliminate such and wrong frame expressed proteins generated in all the probabilities, described above. Thus this method is still valid and forms a valuable protocol in large-scale protein expression and identification.
**4. Overexpression of tissue proteome library and protein purification:** These procedures must be designed based on the vector employed in construction of cDNA libraries. However, overexpression of tissue proteome library employing GJ1158 bacteria (about which it is already discussed earlier) is presented here. Glycerol stocks of the libraries (a, b and c) in GJ1158 bacteria could be inoculated together in LBON with Kanamycin and allowed to grow overnight at 37° C at a constant shaking of 100 rpm. Bacteria sub-cultured on the next day at 1:100 dilutions in LBON with Kanamycin and grown until 600 nm optical density of the culture reached to between 0.6-0.8. Culture should be induced with 300 mM sodium chloride for 3 hours by which time protein expression reaches steady state level, shifted to 4° C and stored for one hour to arrest cell division. Bacteria centrifuged at 5000xg for 10 minutes at 4° C; bacterial pellets recovered and stored at -80° C, till their use for protein purification. Bacteria should be suspended uniformly in 100 mM sodium phosphate buffer, pH 8.0 containing 8 M urea (5 ml buffer / gram wet-weight of bacteria) and cells stirred at room temperature for 60 minutes by gentle vortexing. Suspension was centrifuged at 10,000xg for 30 minutes at room temperature and clear lysate recovered. Clear lysate subjected to chromatography through NiNTA-agarose column employing standard protocols (QIAexpressionist user manual, 2001), for purification of total 6xHis-tagged proteins. Purified protein desalted by thorough dialysis against milliQ water consisting 10 mM EDTA, 3 mM Benzamidine and 1 mM each of PMSF, leupeptin and aprotinin, to prevent possible proteolysis, which precipitates most of the proteins as they are in denatured condition and the protein suspension lyophilized.
**5. Purification of total tissue protein:** Total proteins present in a tissue could be purified employing standard procedures (Vaibhav et al., 2005) prescribed for, 2D PAGE analysis of the tissue protein pools.
**6. Electrophoresis of proteins purified from the Tissue proteome library and the tissue:** Protein purified by NiNTA-agarose column above and lyophilized is dissolved in sample buffer for iso-electrophoresis (IEF) and subjected to IEF and 2D PAGE according to the standard protocols (Joubert-Caron et al., 1999). Standard protein molecular weight markers should also be run in adjacent lanes. Total tissue protein isolated could also be electrophoresed in identical IEF and 2D PAGE gels.
**7. Comparison of proteome library expressed and tissue purified proteins and proteomics analysis:** Successful comparison depends to a large extent on the method employed in the library construction and representation of full-length / truncated cDNAs in the library. Tissues contain many proteins and all their mRNAs. Perhaps because we cloned total tissue cDNAs and expressed all of them, 2D PAGE patterns, of purified proteins from the tissue and its library-expressed protein are expected to be similar. However, few of the protein spots in 2D PAGE profile of library expressed protein might be shifted to lower mass levels, compared to those in the tissue protein profile, because of the occurrence of chosen restriction site sequences, in the middle of such transcripts and truncation of these transcripts during cDNA digestion and cloning into the vector. Library-expressed proteins might also be shifted marginally in their positions on 2D PAGE from tissue proteins, due to their increased mass and changed pI, because of common and extra amino acids present at their N and C termini, which are translated from vector sequences. Protein spots can be dissected from library expressed protein gel, in-gel digested with trypsin, peptides extracted and analyzed by MALDI TOF by standard protocols (Mann et al., 2001). Although in general comparison between the proteins spots expressed in tissue proteome library and with that of the tissue proteins may be possible, one can not expect a one to one comparison between these two patterns. However, comparisons are possible between profiles of proteins expressed in tissue proteome libraries constructed from the tissue of an individual suffering from a disease with that from a normal individual, which possesses interesting and ready applications in medical biotechnology.
**8. Parameters that could cause limitations and lower efficiency of the method:** Levels of individual mRNAs present in a tissue are generally expected to be dependent on their own and their protein turnover rates. However, there could be mRNAs that might associate with protein factors present in the tissue and remain un-translated for various unknown reasons. Thus whether or not the relative proportion of individual proteins present in a tissue is truly proportional to their respective mRNAs present in the same tissue is not known. Thus this method helps only in finding out all the mRNAs present in the tissue in terms of their translated proteins. Although many mRNAs and their respective proteins shall be present in the tissue, whether specific mRNAs of identified proteins present in the tissue get actually translated in the tissue or not needs to be investigated independently.

Several factors like (a) faithful / proportional synthesis of cDNA representing the tissue mRNA pool, (b) efficiency of cDNA ligation into expression vectors (c) efficiency of transformation of plasmids into host bacteria, (d) library induction time required for producing steady state level of protein expression and (e) synthesis of proteases in the bacteria / translated from clones in the library, which could cause degradation of overexpressed proteins are some of the determinants of this technology. During some of the critical steps of protein purification, protein degradation might cause limitations of the total process, particularly when some of the cloned and overexpressed cDNAs are unknown potent proteases. Most of these limitations are either related to normal limitations of library construction procedures and / or dependent on the type of tissue particularly being employed. Thus these have to be standardized for each of the tissues and cannot be described as general precautions.

**In an embodiment of the present invention,** it provides a method to develop a tissue proteome library comprising of the following steps:-
a. isolating the total RNA and mRNA from a tissue,
b. synthesizing total cDNA pool from the mRNA obtained in step a,
c. amplifying recovered cDNA from step b, using primers (containing specific restriction enzyme sites) for incorporating these sites into the amplified cDNAs,
d. digesting PCR amplified cDNAs obtained in step c with the specific restriction enzymes,
e. cloning digested cDNAs obtained in step d, in all three 5'→3' directional frames of an expression vector,
f. transforming the plasmid pool obtained in step e, into bacteria by electroporation, growing the bacteria and induction of the library for protein expression,
g. isolating the expressed proteins in the bacteria obtained from step f,
h. analyzing the expression levels of total proteins obtained from step g,

**In another embodiment of the present invention** wherein the restriction enzyme is a type II restriction enzyme (like *Rsa 1*, *Sfi 1*, *Sgf 1, Pme 1, Not 1* etc) consisting of 5-(or more)-base restriction enzymes that generates cohesive termini upon cleavage.

**In another embodiment of the present invention** the total cDNA library is directionally cloned into expression vectors in either one or all 3 possible frames.

**In another embodiment of the present invention** the library is overexpressed for producing the total library of proteins.

**In yet another embodiment of the present invention,** the pool of cDNA is derived from any biomaterial like viruses, prokaryotic cells, eukaryotic cells, plant cells, insect cells and mammalian tissues, clinical tissue specimen samples, tissue culture grown cells, oocytes, zygotes, embryos and purified cellular organelles, tissue or whole organism.

**In yet another embodiment of the present invention,** cloning a pool of normalized population of cDNAs reverse transcribed from the tissue mRNA pool or released from existing libraries either in one or all three frames of any one or a group of expression vectors.

**In yet another embodiment of the present invention,** cloning / expression of a pool of cDNAs in expression vectors co-cloned and or co-expressed with either protease-inhibitors or protein stabilizing agents.

**In yet another embodiment of the present invention,** a tissue proteome library is prepared, wherein it is useful in making a representative library of the "truly" expressed proteins and overexpressing a large number of transcripts (mRNAs) present in a given tissue.

**In yet another embodiment of the present invention,** the library is useful for overproduction, electrophoretic separation and proteomic analysis / identification of all the proteins present as mRNAs in the tissue.

**In yet another embodiment of the present invention,** the library is useful for identification of novel proteins even if truncated products of cDNAs are cloned and expressed or partially degraded proteins of 10-100 kDa are produced during protein purification from bacteria.

**In yet another embodiment of the present invention,** the library is useful for identification of low abundant and novel proteins / transcripts present in the tissue.

**In yet another embodiment of the present invention,** cDNAs restriction digested with 5 or more nucleotide restriction enzymes from constructed or commercial or pre-made cDNA libraries.

**In yet another embodiment of the present invention,** the library is useful for producing pools of biological molecules like mRNAs / cDNAs / proteins / peptides for research or other purposes like industrial, therapeutic, bio-medical, health-related, biotechnology, food technology and cosmetic purposes including studies and or applications thereof;

**In yet another embodiment of the present invention,** wherein such libraries are useful for analyzing the total cellular protein expression of a tissue selected from the group consisting of expression and or isolation / identification of all or any sub-group of expressed proteins like membrane proteins, DNA-binding proteins, lipoproteins, nuclear proteins, transcription factors, signaling proteins, ribosomal proteins, mitochondrial proteins or any other group of proteins;

**In yet another embodiment of the present invention** the library is used for analysis of expressed protein by chromatography, ID or 2D polyacrylamide gel electrophoresis (PAGE), Iso-electrophoresis (IEF), high performance liquid chromatography (HPLC), Matrix Assisted Laser Desorption-Ionization Time of Flight (MALDI TOF), Electro Spray Ionization Mass Spectrometry (ESI MS).

**In yet another embodiment of the present invention** the library is useful for identification of expressed proteins in said host cell that have a molecular weight of at least 10 kDa.

### The following examples are given by way of illustration of the present invention, which provided the basis and a method to develop a Tissue Proteome Library and therefore these should not be construed to limit the scope of the present invention.

### Example 1

Several mRNAs of proteins were collected from NCBI database and translated in all the 5'→3' directional frames, employing the mRNA / DNA → protein translation tool available at ExPASy Proteomics server; Data of 25 mRNAs, stop codons found in them and computed proteins / peptide masses are summarized in Table 1.

We collected the mRNA sequences of 100 database reported proteins (ranging in size from 10 to 100 kDa) and translated them in all three 5' → 3' directional frames employing the translation tool available at ExPASy proteomics server, we observed that only one of the frames gave a full-length protein. Further, stop codons occurred in the other two incorrect protein-reading frames at an average frequency of 16 to 24 amino acids. This implied that cloning and expression of these cDNAs in incorrect reading frames of expression vectors generates truncated peptides ranging in size between 16-24 amino acids or 1.84 to 2.76 kDa. This is in perfect agreement with the "Theoretical basis for developing a tissue proteome library, by cloning cDNA pool of the tissue in all the three frames of an expression vector", presented above.

When a cDNA is cloned in an incorrect reading frame of an expression vector and proteins expressed, it would produce a polypeptide up to the first stop codon and translation terminated. Since unwanted hypothetical (non-natural) proteins and peptides produced in a randomly cloned and expressed cDNA library is the major bottleneck in developing a tissue proteome library, we decided to analyze a set of mRNAs (expected to produce proteins of masses ranging between 10-100 kDa) collected from NCBI, and for arriving at all the peptides that could be produced from them upon cloning and expression in incorrect reading frames of expression. Information about the mass of the first polypeptides produced from the cDNAs is necessary and sufficient to assess if such wrong frame cloned cDNA could produce hypothetical proteins / peptides under such circumstances. However, so as to gain knowledge about the number of polypeptides of mass values > 10 kDa and mass of the biggest polypeptide generated from the transcripts cloned in incorrect reading frames of expression vectors, we computed this information also from a set of mRNAs and presented it in Table 1. Occasionally transcripts cloned in a tissue proteome library might possess only partial (5'-truncated) cDNAs. They shall give raise to a different set of translation-terminated polypeptides (due to stop codons in the middle of incorrect frame cloned and expressed cDNAs), than those produced from full-length cDNAs. Since we are considering all the polypeptides > 10 kDa (see page 6, paragraph 2, to explain, why we fixed this) arbitrary cut off, this shall validate even cloning and expressing both full-length and 5'-truncated cDNAs in incorrect reading frames of expression vectors, in the tissue proteome library.

Total peptides with mass values > 10 kDa found in the above 25 mRNAs, translated in incorrect frames is 24 out of a total of 1021 peptides formed due to stop codons, which works out to a relative abundance of only 2.35%, while the average number of peptides in protein is about 20 [1021 / (2 x 25) = 20]. Thus such peptides could occur at a rate of one out of five proteins and occurrence of such a peptide in the beginning of a translating cDNA shall be extremely low. As protein synthesis in "incorrect frame expressed cDNAs" is terminated at the first stop codon, the possibility of synthesis of truncated peptides of mass values > 10 kDa shall be extremely low. Further, as genome sequencing of several organisms like *E*. *coli, Mus musculus, Homo sapiens, Drosophilae, c. elegance* are already completed and their gene / protein databases available, even if a few such peptides are produced upon expression of the total library, they can easily be detected as hypothetical proteins from these organisms. In view of the above, few large peptides expressed from transcripts cloned in incorrect (wrong) reading frame for expression shall not be a limitation / shortcoming in developing and expressing tissue proteome libraries. We analyzed 100 mRNAs of proteins recovered from the NCBI database; however, we presented the data only from 25 mRNAs (Table 1) as these data are in concurrence with the total data obtained from all the 100 mRNAs.

### Table 1:

### Data in support of Example 1:

Several cDNAs of proteins were collected from NCBI database and translated in all the 5'→3' directional frames, employing DNA → protein translation tool available at ExPASy proteomics server. Data of 25 cDNAs, stop codons found in them, and proteins / peptide masses are summarized in Table 1:
In the data presented in Table 1, multiple stop codons present in a stretch is considered as a single stop codon. Equal number of stop codons and peptides implies presence of stop codon at the beginning of the sequence in a reading frame. First and largest peptide mass being identical in Table 1 implies that the first is the largest peptide. Only for providing larger opportunity for the occurrence of larger size of peptides and their frequency in wrong frame expressed transcripts, we took the next and immediate peptide into account, when there is a stop codon in the beginning, of a cDNA.

| **Sl. No.** | **Accession & Gene ID Nos.** | **Frame** | **Number of Peptides** | | | **Mass (Da) of the respective peptides** | | **Protein Mass (kDa) and Organism** |
|---|---|---|---|---|---|---|---|---|
| | | | **Stop Codons** | **total** | **>10kDa** | **First** | **Largest** | |
| 1. | NM_001011690 GI: 58696439 | 1 | NIL | NIL | NIL | - | - | 15.77817; RNase A (LOC422633) *Gallus gallus* |
| | | 2 | 3 | 4 | NIL | 797.88 | 9018.41 | |
| | | 3 | 5 | 5 | NIL | 2323.64 | 5550.36 | |
| 2. | NM_007449.2; GI: 112818585 | 1 | NIL | NIL | NIL | - | - | 16.59327; Angiogenin A RNase *Mus musculus* |
| | | 2 | 8 | 9 | NIL | 361.42 | 5253.15 | |
| | | 3 | 6 | 7 | NIL | 2455.64 | 5713.59 | |
| 3. | NM_001013548; GI 61806672 | 1 | NIL | NIL | NIL | - | - | 19.30625; Processing RNase Subunit (*S. cerevisiae*) |
| | | 2 | 10 | 11 | NIL | 3904.48 | 6511.28 | |
| | | 3 | 9 | 10 | NIL | 1633.01 | 6652.42 | |
| 4. | NM_000612.3;c GI: 109148514 | 1 | NIL | NIL | NIL | - | - | 20.14035; Insulin-like growth factor 2, *Homo.sapiens* |
| | | 2 | 2 | 3 | One | 6081.75 | 10069.51 | |
| | | 3 | 4 | 5 | NIL | 2882.30 | 6614.30 | |
| 5. | NM_000800.1: GI: 10834983 | 1 | NIL | NIL | NIL | - | - | 23.71822; Interleukin 6 (Interferon, beta 2), *Homo sapiens* |
| | | 2 | 13 | 13 | NIL | 4096.80 | 4096.80 | |
| | | 3 | 11 | 12 | NIL | 5828.90 | 5828.90 | |
| 6. | NM_001046833.1; GI: 114051450 | 1 | NIL | NIL | NIL | - | - | 29879.91; E2F Transcription factor 4-like protein, *B. mori* |
| | | 2 | 16 | 16 | NIL | 244.33 | 4560.14 | |
| | | 3 | 27 | 28 | NIL | 132.09 | 2880.46 | |
| 7. | AB234211 GI: 101919348 | 1 | NIL | NIL | NIL | - | - | 30.06927; Ipomoea nil Transcriptional regulator, |
| | | 2 | 15 | 16 | NIL | 2028.40 | 5547.28 | |
| | | 3 | 14 | 15 | NIL | 75.0666 | 6711.46 | |
| 8. | NM_053056.2; GI: 77628152 | 1 | NIL | NIL | NIL | - | - | 33.72911; Cyclin D1 (CCND1), *Homo sapiens* |
| | | 2 | 11 | 12 | NIL | 3191.65 | 6948.00 | |
| | | 3 | 3 | 4 | One | 11037.4 | 11037.4 | |
| 9. | NM_011098.3; GI: 109948273 | 1 | NIL | NIL | NIL | - | - | 35.32066; Paired like Homeodomain Transcription factor 2; *Mus musculus* |
| | | 2 | 12 | 13 | One | 1141.30 | 19523.55 | |
| | | 3 | 10 | 11 | NIL | 2373.61 | 8020.97 | |
| 10. | MM_000041.2; GI: 48762938 | 1 | NIL | NIL | NIL | - | - | 36.15408; Apolipoprotein E, *Homo sapiens* |
| | | 2 | 7 | 7 | One | 8766.02 | 16179.61 | |
| | | 3 | 3 | 4 | One | 5666.27 | 14034.51 | |
| 11. | MM_0010464531; GI: 111053106 | 1 | NIL | NIL | NIL | - | - | 38.76669; Pre-B-cell leukemia transcription factor 3 (PBX3); *Bos taurus* |
| | | 2 | 11 | 12 | NIL | 1503.77 | 6922.07 | |
| | | 3 | 15 | 16 | One | 9002.80 | 10072.08 | |
| 12. | NM 177989; GI: 98985780 | 1 | NIL | NIL | NIL | - | - | 43.23628; Actin-like 6A, Transcript 2, *Homo sapiens* |
| | | 2 | 22 | 23 | NIL | 390.43 | 9320.48 | |
| | | 3 | 22 | 23 | NIL | 575.69 | 6641.3 | |
| 13. | NM_008091.2; GI: 40254638 | 1 | NIL | NIL | NIL | - | - | 47.96792; GATA vbinding protein 3 *Mus musculus* |
| | | 2 | 12 | 13 | NIL | 361.42 | 9818.54 | |
| | | 3 | 10 | 11 | One | 4864.32 | 13986.06 | |
| 14. | NM_000616.3; GI: 91992151 | 1 | NIL | NIL | NIL | - | - | 51.11054; CD4 molecule, *Homo sapiens* |
| | | 2 | 27 | 27 | NIL | 4492.16 | 6085.10 | |
| | | 3 | 22 | 23 | NIL | 631.65 | 6887.71 | |
| 15. | NM_004624; GI: 15619005 | 1 | NIL | NIL | NIL | - | - | 51.54717; Vasoactive intestinal peptide receptor 1, *Homo sapiens* |
| | | 2 | 14 | 15 | One | 4509.36 | 11154.36 | |
| | | 3 | 13 | 14 | NIL | 3530.11 | 9302.73 | |
| 16. | NM_001046556.1; GI: 114053148 | 1 | NIL | NIL | NIL | - | - | 55.70851; Transcription factor 2; (MTF2), *Bos Taurus* |
| | | 2 | 20 | 21 | NIL | 4489.57 | 8947.38 | |
| | | 3 | 36 | 37 | NIL | 1703.94 | 6802.05 | |
| 17. | NM 018191.3; GI: 857242758 | 1 | NIL | NIL | NIL | - | - | 58.252.24; Chromosome Condensation regulator *Homo sapiens* |
| | | 2 | 25 | 26 | NIL | 5463.39 | 6274.42 | |
| | | 3 | 27 | 28 | NIL | 3468.97 | 8334.72 | |
| 18. | NM_001046150.1; GI: 114052005 | 1 | 30 | NIL | NIL | - | - | 59.16321; Similar to transcri-ption factor TFIIH *Bos Taurus* |
| | | 2 | 30 | 31 | One | 6848.08 | 10378.30 | |
| | | 3 | 36 | 37 | NIL | 850.00 | 5748.34 | |
| 19. | NM_022659.2; GI: 113930702 | 1 | NIL | NIL | NIL | - | - | 62.64998; Early B-cell factor 2 (EBF2), *Homo sapiens* |
| | | 2 | 23 | 24 | One | 865.09 | 10284.48 | |
| | | 3 | 24 | 25 | One | 4203.63 | 12776.82 | |
| 20. | NM_138942.3; GI: 110815860 | 1 | NIL | NIL | NIL | - | - | 70.31400; Dopamine beta hydroxylase; *Mus musculus* |
| | | 2 | 12 | 13 | One | 6701.74 | 10512.36 | |
| | | 3 | 25 | 26 | One | 1975.23 | 17587.42 | |
| 21. | NM_011882; GI: 31982957 | 1 | NIL | NIL | NIL | - | - | 83.27496; Ribonuclease L, *Mus musculus* |
| | | 2 | 42 | 43 | NIL | 3910.46 | 7338.61 | |
| | | 3 | 54 | 55 | NIL | 4366.02 | 6792.45 | |
| 22. | NM_031982; GI: 14010883 | 1 | NIL | NIL | NIL | - | - | 94.94814;Transient receptor potential Cation channel; *Rattus norvegicus* |
| | | 2 | 26 | 27 | One | 673.72 | 11546.94 | |
| | | 3 | 36 | 37 | NIL | 334.33 | 5851.69 | |
| 23. | NM_008806.2; GI: 113930734 | 1 | NIL | NIL | NIL | - | - | 98.53502; Phosphodieste-rase 6B, (Pde6b), *Mus musculus* |
| | | 2 | 40 | 40 | NIL | 2779.19 | 8318.82 | |
| | | 3 | 50 | 51 | NIL | 371.37 | 7667.45 | |
| 24. | NM_000926.3; GI: 110611913 | 1 | NIL | NIL | NIL | - | - | 98.98114;Progestero ne receptor (PGR); *Homo sapiens* |
| | | 2 | 35 | 35 | Two | 218.25 | 17910.92 | |
| | | 3 | 35 | 36 | Two | 132.09 | 19982.02 | |
| 25. | NM_001042413.1; GI: 109637783 | 1 | NIL | NIL | NIL | - | - | 99.60490; GLIS family zinc finger Transcript variant1, *Homo sapiens* |
| | | 2 | 20 | 20 | Three | 671.70 | 17197.87 | |
| | | 3 | 26 | 27 | Three | 2106.30 | 27906.84 | |

### Example 2:

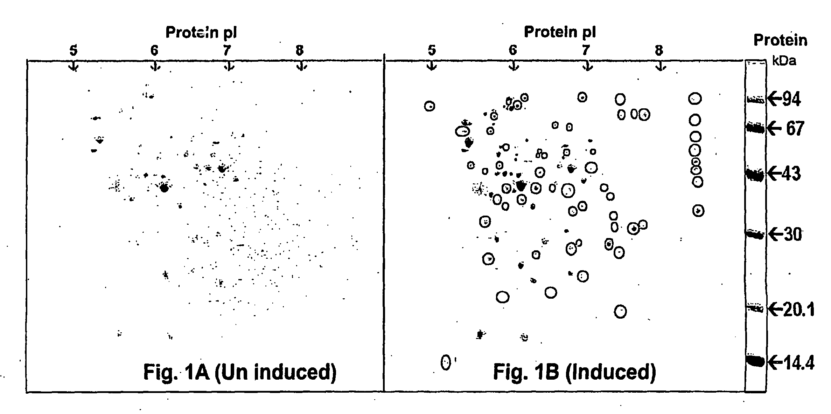

Taking clue from 'Example 1" above, we performed an interesting experiment; Employing the "materials and methods" elaborated in "Example 3" below, we synthesized total cDNAs from purified snake oocyte mRNA, and cloned the recovered cDNA pool in pT7T3D directional cloning vector. We transformed the plasmid pool into salt inducible GJ1158 bacteria by electroporation and expressed the total library of transcripts. About 33% of the cDNAs-cloned shall be in the correct reading frame of the vector for expression and shall produce true proteins, while other cDNAs cloned in incorrect reading frames shall produce truncated peptides. If bacteria were capable of producing a total library of proteins from such a library upon expression, we would expect that expressed proteins should range in their iso-electric point (pI) and mass (kDa) similar to the bacterial proteins. Analysis of total proteins of the 'un-induced' and 'induced' library on a 12% 2D PAGE (Fig. 1A and Fig. 1B), was performed and protein profiles compared. Comparison of the pattern of the protein spots in the "Induced" with that of the "Un-induced" in Fig. 1, clearly shows that the spread of expressed protein in the range of pI and mass (shown by purple circles in the 2D protein pattern) matches very well with that of the rest of the (*E. coli*) proteins. This clearly demonstrates that bacteria are capable of expressing a library of proteins, when cloned and induced together in an expression vector. Further bacteria do not appear to discriminate between their and cloned transcript proteins and exhibits no size bias in protein expression. However, we are very much aware that our library would express only one third of the transcripts cloned and expressed in correct reading expression frames of the library into full-length proteins. From the above results, we predicted that total cDNA library could be directionally cloned into expression vectors in all three possible frames of expression of a vector and such a library could be overexpressed for producing the total library of proteins, which we designated as "the Tissue Proteome Library".

When we clone and overexpress a single cDNA in the correct frame of an expression vector and resolve 200 µg of total induced protein in such a gel, it is expected to give a huge single protein spot expressed from the cloned cDNA amounting to about 30-40% of the total protein resolved in the gel. However, when a library of cDNAs is cloned and expressed and 200 µg of protein is resolved, only about 10-13% (one third) of recombinant protein expressed in the correct frames shall emerge as multiple protein spots, while remaining 20-27% of the in-correct frame expressed proteins shall end up as peptides and run off the gel due to their low masses. Presence of a few major protein spots of identical intensity in the "Un-induced" and "Induced' gels (Fig. 1), agrees with the fact that we resolved identical amounts of protein in these gels. This clearly demonstrated that bacteria are capable of expressing a library of cDNAs and snake oocyte cDNA expression library is able to express a full range of proteins cloned in the correct frames for expression.

### Table 2

### DNA-Binding Proteins Identified by the Proteomics Analysis:

Column 1: Serial number; 2: SSP number; pI and Protein Mass (kDa), in order; 3: Search tool used; Protein / Estd Z score, in order; 4: Protein, pI & Mass (kDa), organism, and gene Id in order; and Column 5: Match % of sequence and peptides.

| **1** | **2** | **3** | **4** | **5** |
|---|---|---|---|---|
| 1. | 1304 5.30 16.20 | Profound # 1.48 (*) | Unnamed protein product; 5.60; 149.71; [*Tetraodon nigroviridis*]; **gi\|47219343** | 9; 13 / 25 |
| | | Mascot 75 / 76 (*) | Unnamed protein product; 5.63; 149.631; [*Tetreodon nigrovitidis*]; **gi\|47219343** | 10; 14 / 25 |
| 2. | 1305 5.40 16.00 | Profound 2.43 | 30S ribosomal protein S6; 5.20; 15.17; [*Shigella flexneri 2a str. 301*]; **gi\|24054885** | 27; 6 / 41 |
| | | Profound 1.26 (*) | Signal transduction histidine kinase; 5.20; 49.82; [*Rubrivivax gelatinosus PM1*]; **gi\|47573640** | 27; 10 / 41 |
| | | Mascot 76 / 76 | Signal transduction histidine kinase; 5.18; 49.803; [*Rubrivivax gelatinosus PM1*]; **gi\|47673640** | 20; 9 / 41 |
| 3. | 1405 5.30 22.70 | Profound ø 2.43 | Another partner for ARF 1; 5.80; 52.19; [*Mus musculus*]; **gi\|18204847** | 21; 7 / 25 |
| | | Mascot 70 / 77 (*) | Another partner for ARF 1; 5.80; 52.176; [*Mus musculus*]; **gi\|21450221**(Sequence same as gi\|18204847) | 26; 9 / 25 |
| 4. | 1701 5.30 36.50 | Profound 2.43 | Chain B, Structure of Transaldolase B; 5.10; 35.07; [*E. coli*]; **gi\|1941983** | 34; 9 / 53 |
| | | Mascot 58 / 70 (*) | Hypothetical protein ebA4606; 6.37; 43.471; [*Azoarcus sp. EbN1*]; **gi\|56478047** | 46; 15 / 53; |
| 5. | 1955 540 40.00 | Profound ø 2.43 | Vim1 protein; 5.10; 52.84; [*Xenopus laevis*]; **gi\|38303787** | 35; 12 / 52 |
| | | Mascot 45 / 76 (*) | LOC496727 protein; 5.24; 55.483; [*Xenopus tropicalis*]; **gi\|56789060** (Sequence same as gi\|38303787) | 27; 11 / 52 |
| 6. | 2203 5.60 15.20 | Profound ø 2.43 | PREDICTED: similar to HNRPC protein; 4.88; 26.822; [*Homo sapiens*]; **gi\|51468879** | 33; 7 / 19 |
| | | Mascot 47 / 76 (*) | PREDICTED: similar to HNRPC protein; 4.88; 26.822; [*Homo sapiens*]; **gi\|51468879** | 33; 7 / 19 |
| 7. | 2302 5.50 15.90 | Profound ø 2.43 | CAA303719.1 protein; 9.35; 51.50; [*Oryza sativa*]; **gi\|5777831** | 24; 14 / 46 |
| | | Mascot 64 / 76 (*) | CAA303719.1 protein; 9.35; 51.481; [*Oryza sativa*]; **gi\|5777631** | 24; 14 / 46; |
| 8. | 3205 6.10 15.30 | Profound 2.43 | DNA-binding protein H-NS; 5.40; 15.52; [*E. coli O157:H7*]; **gi\|15830993** | 34; 7 / 46 |
| | | Mascot 60 / 76 (*) | DNA-binding protein HLP-II (HU, BH2, HD, NS); pleiotropic regulator; 5.43;15.53;[*Shigella flexneri 2a str 301*]; **gi\|24112633;** (Sequence same as gi\|15830993) | 62; 8 / 46 |
| 9. | 3601 6.00 31.00 | Profound 2.43 | Single-stranded DNA-binding protein; 5.4; 18.79; [*Serratia marcescens*]; **gi\|47270** | 48; 6 / 14 |
| | | Mascot 55 / 77 (*) | Single-stranded DNA-binding protein; 5.44; 18.787; [*Serratia marcescens*]; **gi\|47270** | 48; 6 / 14 |
| 10. | 4401 6.20 22.70 | Profound 1.37 (*) | ssDNA-binding protein; 5.5; 18.963; [*Shigella flexneri 2a str. 301*]; **gi\|56394074** | 21; 4 / 25 |
| | | Mascot 28 / 57 (*) | ssDNA-binding protein; 5.44; 18.963; [*Shigella flexneri 2a str. 301*]; **gi\|56480523** (Sequence same as gi\|56394074) | 20; 4 / 25 |
| 11. | 4402 6.20 21.10 | Profound 2.43 | ssDNA-binding protein; 5.40; 18.83; [*E. coli O157:H7 EDL933*]; **gi\|15804651** | 44; 9 / 47 |
| | | Mascot 68 / 76 (*) | Single-strand DNA-binding protein (ssb) 18.993; 5.44; [*E. coli*]; **gi\|147870** (Sequence same as gi\|15804651) | 43; 10 / 47 |
| 12. | 4955 6.40 >90 | Profound # 1.99 | DNA helicase related protein; 6.40; 152.14; [*Pyroccocus abyssi GE5*]; **gi\|14520327** | 16; 15 / 22 |
| | | Mascot 57 / 76 (*) | DNA replication helicase, dna2 homolog; 6.36; 152.063; [*Pyrococcus abyssi*]; **gi\|5457542** (Sequence same as gi\|14520327) | 16; 15 / 22 |
| 13. | 5304 6.70 18.10 | Profound ø 1.58 (*) | DNA polymerase, bacteriophage-type; 6.80; 22.70; [*Archaeoglobus fulgidus DSM 4304*]; **gi\|2648243** | 27; 6 / 19 |
| | | Mascot 54 / 76 (*) | DNA polymerase, bacteriophage-type; 6.75; 22.74; [*Archaeolobus fulgidus DSM 4304*]; **gi\|11499858** (Sequence same as gi\|2648243) | 27; 6 / 19 |
| 14. | 5602 6.80 30.50 | Profound # 1.58 (*) | DNA replication and repair protein 8.84; 40.72; [*Erwinia carotovora subsp. atroseptica SCRI1043*]; **gi\|50123357** | 24; 9 / 26 |
| | | Mascot 51 / 76 (*) | DNA replication & repair protein; 8.84; 40.711; [*Erwinia carotovora subsp. atroseptica SCRI1043*]; **gi\|50123357** | 24; 9 / 26 |
| 15. | 5702 6.50 34.10 | Profound 2.13 | Hypothetical protein DKFZp5660224.1 - human; 5.50; 36.28; [*Homo sapiens*]; **gi\|11360197** | 37; 13 / 41 |
| | | Mascot 88 / 76 | Hypothetical protein DKFZp5660224.1 - human; 5.55; 36.276; [*Homo sapiens*]; **gi\|11360197** | 36; 12 / 41 |
| 16. | 6403 7.50 21.40 | Profound 2.43 | Hypothetical protein S1573; 6.80; 19.99; [*Shigella flexneri 2a str. 2457T*]; **gi\|30041221** | 55; 7 / 24 |
| | | Mascot 56 / 78 (*) | Hypothetical protein ydjA; 6.84; 19.992; [*Shigella flexneri Q7UCJ8_SHIFL*] **gi\|24112838** (Sequence same as gi\|30041221) | 55; 7 / 24 |
| 17. | 6602 7.00 30.50 | Profound 2.43 | Chain A, Crystal Structure Of Exfoliative Toxin B; 6.30; 27.17; [*Staphylococcus Aureus*]; **gi\|28373263** | 38; 6 / 46 |
| | | Mascot 60 / 76 (*) | Amino acid transport ATP-binding protein; 5.92; 30.14; [*Streptococcus pyogenes MGAS10394*]; **gi\|50913613** | 58; 12 / 46 |
| 18. | 7303 >8.00 16.60 | Profound ø 1.76 | ATPases involved in chromosome partitioning; 9.30; 20.55; [*Crocosphaera watsonii WH 8501*]; **gi\|45525746** | 37; 7 / 33; |
| | | Mascot 54 / 77 (*) | Similar to ATPases involved in chromosome partitioning; 9.45; 21.607; [*Crocosphaera watsonii WH 8501*]; **gi\|67922927** (Sequence same as gil45525746) | 25; 7 / 33 |
| 19. | 7305 >8.00 18.00 | Profound 2.43 | Histone-like protein, located in outer membrane or nucleoid: 9.7; 17.67; [*Shigella flexneri 2a str. 301*]; **gi\|24050382** | 36; 11 / 42 |
| | | Mascot 79 / 76 | Histone-like protein; 9.69; 17.677; [*Shigella flexneri 2a str. 301*]; **gi\|24111613;** (Sequence same as gi\|24050382) | 45; 11 / 42 |
| 20. | 7702 >8.00 43.70 | Profound 2.43 | Similar to cell division protein RodA, FtsW; 8.40; 43.68; [*Listeria monocytogenes EGD-e*]; **gi\|16804465** | 27; 7 / 19 |
| | | Mascot 65 / 76 (*) | Hypothetical protein Imo2427; 8.41; 43.69; [*Listeria monocytogenes EGD-e*]; **gi\|16804465** | 27; 7 / 19 |
| 21. | 7901 8.00 50.00 | Profound ø 1.83, | Unnamed protein product; 6.30; 71.46; [*Mus musculus*]; **gi\|26349029** | 15; 11 / 30 |
| | | Mascot 60 / 62 (*) | Zinc finger protein 398; 6.25; 71.434; [*Mus musculus*]; **gi\|27819692** (Sequence same as gi\|26349029) | 15; 12 / 30 |
| 22. | 7905 >8.00 66.00 | Profound ø 1.63 (*) | Putative DNA glycosylase; 9.31; 67.43; [*Mus musculus*]; **gi\|19353217** | 25; 12 / 36 |
| | | Mascot 44 / 76 (*) | Putative DNA glycosylase; 9.31; 67.406; [*Mus musculus*]; **gi\|19353217** | 30; 17 / 36 |
| 23. | 8001 > 8.00 52.81 | Profound # 1.68 | Predicted-similar to SWI/SNF-related matrix-associated actin-dependent regulator of chromatin A (SMARCHA)-like HepA-related protein 1; 9.60; 119.98; [*Gallus gallus*]; **gi\|50750628** | 19; 13 / 25 |
| | | Mascot 58 / 77 (*) | Predicted-similar to SWI/SNF-related matrix-associated actin-dependent regulator of chromatin A (SMARCHA)-like HepA-related protein 1; 9.30; 119.92; [*Gallus gallus*]; **gi\|50750628** | 18; 13 / 25 |
| 24. | 8004 > 8.00 83.20 | Profound # 2.43 | LINE-1 reverse transcriptase homolog; 9.80; 147.10; [Homo sapiens]; **gi\|126295** | 21; 20 / 31 |
| | | Mascot 64 / 77 (*) | LINE-1 reverse transcriptase homolog; [Homo sapiens]; 9.67; 147.02; **gi\|126295** | 23; 21 / 31 |

| | | | | |
|---|---|---|---|---|
| Notes: Ø. Clue about protein mass, pl & organism of best score from Mascot were used in Profound #. Clue about protein mass and pI of best score from Mascot were used in Profound Φ. Clue about the organism of best score from Mascot was used in Profound. (*) Indicates that Protein / Estd Z score is somewhat lower than 'Significant score level' | | | | |

### Example 3:

Encouraged by the above findings that bacteria are able to express a library of proteins, we attempted and succeeded in isolation of a rear and low abundant group of proteins (DNA-binding Proteins) from snake oocyte cDNA library expressed proteins and characterizing them, employing the proteomics approach. We expected that such an exercise should form the testimony and basis for successful development of "a tissue proteome library". DNA-binding proteins expressed in snake oocyte cDNA library form only a very small fraction of the tissue proteins. Since these are low abundant proteins, we certainly require large amounts of the library-expressed protein. We grew 90 L of bacterial culture in batches of 10 L and induced the cultures for protein expression at appropriate level of culture growth (optical density). Bacteria were harvested, homogenized in appropriate native buffer condition in the presence of protease inhibitors and soluble protein saved. Insoluble protein was dissolved in denaturing solvents, refolded and total protein recovered almost quantitatively in the soluble form. Total soluble protein was subjected to chromatography through snake DNA-Sepharose column for isolation of the DNA-binding proteins. We resolved the recovered DNA-binding proteins on 12% 2D PAGE and subjected protein spots to analysis by proteomics approach for protein identification. Interestingly we recovered several snake DNA-binding proteins. Further, isolated DNA-binding proteins were 1.80 mg out of 450 mg of total library protein, which works out to 0.40% of total library protein. Amount of DNA-binding protein recovered is in agreement with the expected recovery of DNA-binding proteins from the tissue proteins. Methods employed and the results obtained in our study regarding cloning of total snake oocyte cDNA pool, overexpression of total library of proteins, purification of the DNA-binding proteins and their identification employing the proteomics approach are given below.

**Table 3**

| **Snake oocyte DNA-binding proteins identified with available sequence motifs and their predicted physiological function:** | | | | |
|---|---|---|---|---|
| **Sl. No.** | **SSP No.** | **Protein, gene and organism of identified protein** | **Domain similarity with other known proteins** | **Functional predicted with respect to available domains** |
| 1. | 1304 | Unnamed protein product; [*Tetraodon nigroviridis*]; gi\|47219343 | immunoglobulin domain; Tyrosine kinase, catalytic domain & Serine/ Threonine protein kinases, catalytic domains | Signal transduction Mechanisms; Transcription, DNA replication, recombination, and repair |
| 2. | 1305 | 30S ribosomal protein S6; [*Shigella flexneri 2a str. 301*]; gi\|24054885 | Ribosomal S6; RpsF; | Translation; ribosomal structure and biogenesis |
| | | Signal transduction histidine kinase; [*Rubrivivax gelatinosus PM1*]; gi\|47573640 | PAS; His Kinase A; HATPase c; | Transcription / Signal transduction mechanisms; Cell motility and secretion |
| 3. | 1405 | Another partner for ARF 1; [*Mus musculus*]; gi\|18204847 | Zinc Finger domain; Putative transcriptional repressor regulating G2/M transition | Transcription / Cell division and chromosome partitioning |
| 4. | 1701 | Chain B, Transaldolase B; [*Azoarcus sp. EbN1*]; gi\|56478047 | Transaldolases TalB | Non-oxidative (pentose phosphate pathway) carbohydrate metabolism |
| | | Hypothetical protein ebA4606; [*Azoarcus sp. EbN1*]; gi\|56478047 | Archaeal ATPase; Holliday junction resolvasome, helicase subunit | DNA replication, recombination and repair |
| 5. | 1955 | Vim1 protein; [*Xenopus laevis*]gi\|36303787 | intermediate filament proteins consisting of Intermediate filament head (DNA binding) and Intermediate filament domains | Chromosome segregation ATPases involved in Cell division and chromosome partitioning |
| | | LOC496727 protein; [*Xenopus tropicalis*]; gi\|56789060 | | |
| 6. | 2203 | Predicted similar to HNRPC protein; [*Homo sapiens*]: gi\|51468879 | RRM; RNA recognition motif | Nuclear pre-mRNA processing |
| 7. | 2302 | CAA03719.1 protein; [*Oryza sativa*]; gi\|5777631 | Tub family protein | Not known |
| 8. | 3205 | DNA-binding protein H-NS; [*E. coli*-*O157*: *H*7]; gi\|15830993 | Histone-like proteins containing HNS domain | DNA-binding |
| 9. | 3601 | Single-stranded DNA-binding protein; [*Serratia*. *marcescens*]; gi\|147270 | Ssb (Single-stranded DNA-binding); PriB (Primosomal replication) domains | DNA replication, recombination, and repair |
| 10. | 4401 | ssDNA-binding protein; [*Shigella flexneri 2a str. 301*]; gi\|56384074 | Primosomal replication protein N (PriB). PriB forms a complex with PriA, PriC & ssDNA | DNA replication, recombination and repair |
| 11. | 4402 | ssDNA-binding protein; [*E. coli O157:H7 EDL993*]; gi\|15804651 | Single Stranded Binding domain | DNA replication, recombination DNA replication, recombination and repair |
| 12. | 4955 | DNA helicase related protein; [*Pyrococcus abyssi GE5*]; gi\|14520327 | RecB nuclease; Super family; DNA & RNA helicases; ATP-dependent exo DNAses | Predicted nuclease (RecB family) protein |
| | | DNA replication helicase; [*Pyrococcus abyssi*]; gi\|5457542 | RecB nuclease; Super family I DNA and RNA helicases; ATP-dependent exo DNAses | DNA replication helicase, dna2 homolog |
| 13. | 5304 | DNA polymerase, bacteriophage-type; [*Archaeoglobus fulgidus* DSM 4304]; gi\|264243 | Uracil-DNA glycosylase domain | DNA replication, recombination and repair |
| 14. | 5602 | DNA replication & repair protein; [*Erwinia carotovora subsp*. *atroseptica SCRI1043*]; gi\|50123357 | Two domains of "structural maintenance of chromosome" (SMC_N) | DNA replication recom-bination, & repair; Cell division & chromosome partitioning |
| 15. | 5702 | Glial fibrillary acidic protein; [*Homo sapiens*]; gi\|27695487; | Intermediate filament homologous to Smc (structural maintenance of chromosome) ATPases, Zn-ribbon protein and DNA repair ATPase | Cell division & chromosome partitioning; nucleic add-binding; DNA replication, recombination and repair |
| 16. | 6602 | Exfoliative Toxin B; [*Staphylococcus aureus*]; gi\|28373263 | Typically periplasmic protease; C-terminal PDZ domain and DegQ domain | Trypsin-like serine proteases; Posttranslational modification, protein turnover, chaperones |
| | | ABC type ATPase-nucleotide binding protein; [*Streptococcus pyogenes MGAS10394*]; gi\|50913613 | Nucleotide binding domain, signature motif Q-loop, H-loop/switch region; Walker A & B motif/P-loop, found in ATP / GTP-binding and hydrolyzing proteins. | Transport of various components like AA, peptides, metal ions, drugs & vitamins; membrane fusion, proteolysis; DNA replication and repair; |
| 17. | 7303 | Similar to ATPases involved in chromosome partitioning; [*Crocosphaera watsonii WH 8501*]; gi\|67922927 | SRP54-type protein, with GTPase domain and ParA family ATPase; | Cell division and chromosome partitioning; Intracellular trafficking and secretion |
| 18. | 7305 | Histon-like protein; [*Shigella flexneri 2a str*. *301*]; gi\|24050382 | Histone-like proteins containing HNS domain and OmpH-like | Cell envelope biogenesis, outer membrane |
| 19. | 7702 | Hypothetical protein Imo2427; [*Listeria monocytogenes EGD-e*]; gi\|16804465 | Cell cycle protein containing FtsW_RodA_SpoVE domain | Not known |
| 20. | 7901 | Zinc finger protein 398; [*Mus musculus*]; gi\|26349029 | KRAB-krueppel associated box COG5048, FOG: Zn-finger domain | DNA replication, recombination, & repair; Post-translational modification, protein turnover, chaperones; |
| 21. | 7905 | Putative DNA glycosylase: [Mus musculus]; gi\|19353217 | GRF zinc finger; Formamidopyrimidine-DNA glycosylase H2TH domain; | DNA repair enzyme excising oxidised purines from damaged DNA, DNA replication, recombination & repair |
| 22. | 8001 | Predicted similar to SWI/SNF-related matrix-associated actin-dependent regulator of chromatin A (SMARCA)-like Hepa-related protein 1; [*Gallus gallus*];gi\|50750628 | Helicase superfamily c-terminal domain; associated with DEXDc-, DEAD-, & DEAH-box proteins, yeast initiation factor 4A, Ski2p, & Hepatitis C virus NS3 helicases | A diverse family of proteins involved in ATP-dependent DNA or RNA unwinding |
| 23. | 8004 | LINE-1 reverse transcriptase homolog; [*Homo sapiens*]; gi\|126295 | Endo / Exo nuclease / phosphatase; RNA-dependent DNA polymerase; Exonuclease III; Metal-dependent hydrolase & Chromosome segregation ATPase | Cell division and chromosome partitioning; DNA replication, recombination, and repair |

### Methodology employed in construction of snake oocyte cDNA library, expression and isolation of DNA-binding proteins from the library expressed protein:

Materials: Electrophoresis chemicals- acrylamide, bis-acrylamide, TEMED, coomassie blue R-250, 2-mercaptoethanol, dithiothreitol and reagents like trizol, urea, guanidine hydrochloride and SDS were obtained from Sigma Chemical Company, St. Louis, MO, (USA). Protease inhibitors- PMSF, leupeptin, benzamidine and aprotinin were purchased from Boehringer Mannheim, Germany. CNBr-activated Sepharose, TimeSaver cDNA synthesis kit, directional cloning toolbox and PT7T3D cloning vector were ordered from Pharmacia Biotech (Sweden). Ready to use iso-electrophoresis strips (IPG strips) were purchased from BIO-RAD Laboratories (USA). Dyna beads mRNA purification kit was purchased from Dynal, USA. Other reagents of analytical grade were procured from local suppliers, Qualigens, E. Merck and BDH.

### Methods:

**a. Collection of snake oocytes:** Oocytes were collected from rat snakes *(Ptyas mucosus)* during their breeding season, flash frozen in liquid nitrogen and stored at -80° C till their use.
**b. Construction of snake-oocyte cDNA library:** Total RNA was isolated from snake oocytes following Trizol extraction method (Chomczynski and Sacchi, 1987). In brief 1 g of oocyte tissue was homogenized in 10 ml of commercial Trizol reagent containing 0.2 ml/ml of chloroform. Chloroform efficiently denatures proteins and yields RNA free of protein contamination. RNA was precipitated from aqueous phase with iso-propanol (0.5 ml/ml) and desalted by repeated washing with 70% ethanol. Polyadenylated (poly-A)-RNA (mRNA) was purified from total RNA, using Dyna beads mRNA purification system. Poly-A RNA was converted to cDNA using Time-saver cDNA synthesis kit and purified cDNA fragments consisting of 0.5-4.5 kb size were ligated into PT7T3D phagemid directional cloning vector between *EcoR1* and *Not1* restriction sites, according to the manufacturer's protocols. Resulting recombinant phagemids were transformed into GJ1158, a salt inducible strain of *E. coli* (Bhandari and Gowrishankar, 1997) by electroporation. Isolated mRNA was intact and cDNA syntheses gave transcripts between 0.5-4.5 kb, which are expected to produce proteins of a wide range in molecular weight.
**c. Induction of snake oocyte cDNA library:** Glycerol stocks of the library and control GJ1158 bacteria were inoculated in LBON (Luria Broth, containing no NaCl) medium with ampicillin and allowed to grow overnight at 37° C at a constant shaking of 100 rpm. Next day bacteria were sub-cultured at 1:100 dilutions in LBON with ampicillin and grown until 600 nm optical density (OD) of the culture reached to about 0.6-0.8 (Bhandari and Gowrishankar, 1997). Culture was then induced with 300 mM sodium chloride for 3 hrs and un-induced culture was also grown for 3 hrs under identical conditions. The cultures were shifted to 4° C and stored for one hour to arrest cell division. This was followed by centrifugation at 5000xg for 10 min at 4° C and bacterial pellets obtained were stored at -80° C, till their use for protein purification.
**d. Protein extraction from bacterial pellets:** Bacterial pellet (12 g) from 2 L culture was uniformly suspended in 40 ml of 50 mM Tris-HCl, pH 8.0 containing 0.1% Triton X-100, 100 µg/ml lysozyme, 3 mM benzamidine and 1 mM each of EDTA, PMSF, aprotinin and leupeptin, and incubated for one hour at room temperature. The suspension was thoroughly sonicated at 4° C for lysis of bacteria, shearing bacterial DNA and lowering solution viscosity, and soluble protein recovered by centrifugation at 10,000xg for 30 min at 4° C. Sediment was subjected to protein refolding (Anderson et al., 1999) by dissolving in 20 ml of 50 mM Tris-HCl, pH 8.0 containing 8 M Guanidine hydrochloride + 1 mM DTT and dialyzing for 12 and 4 hours successively against 50 mM Tris-HCl, pH 8.0 containing 5 mM cysteine and 15 mM cystine and 2 M urea and the same medium without urea. Dialyzed protein was centrifuged as above and insoluble protein subjected to one more cycle of refolding process. Supernatants of refolded proteins were combined with earlier saved soluble protein and subjected to chromatography through snake DNA-Sepharose.
**e. Preparation of DNA-Sepharose affinity matrix:** DNA-Sepharose affinity material was prepared employing standard procedures, by coupling *Hinf1* digested female snake (*Ptyas mucosus*) genomic DNA to CNBr-activated Sepharose (Pharmacia) as per the manufacturers protocols. High molecular weight genomic DNA was isolated from kidney or liver of female snake, employing standard protocol (Kodanaga and Tjian, 1986) and 100 µg of DNA was used per ml of Sepharose for coupling.
**f. Affinity purification of DNA-binding proteins:** Total protein extracted and pooled from 2 L of bacterial culture was subjected to chromatography through a 25 ml snake DNA-Sepharose column. Unbound protein was recycled 2-3 times through the column to provide greater opportunity for binding of snake proteins and enhancing recovery of the bound protein. Loosely bound protein was washed off the column with 50 column volumes of 50 mM Tris-HCl, pH 8.0, containing 1 mM EDTA, 1 mM DTT and 100 mM NaCl, followed by washing the column with 50 more column volumes of the same buffer, but containing 300 mM instead of 100 mM NaCl. DNA-binding proteins were eluted from the column with 1 M NaCl in the same buffer. Eluted protein was concentrated using Amicon filtration with 10-kDa cut-off membranes followed by thorough dialysis against 100 volumes each time with several changes of 50 mM, Tris-HCl pH 8.0, with 3 mM benzamidine and 1 mM each of PMSF, EDTA, leupeptin and aprotinin. Dialyzed protein was concentrated using speed-vac concentrator and dissolved in the sample buffer for IEF.
**g. 1D and 2D PAGE, gel imaging and image analysis:** One-dimensional SDS PAGE of proteins was performed in 12.5% SDS polyacrylamide gels using standard protocol (Laemmli, 1970). Standard protein markers were also run in adjacent lane for assessing molecular weight of resolved proteins. 2D PAGE was conducted according to standard protocols (Joubert-Carton et al., 1999 & O'Farrell, 1975). First dimension was IEF, run in IPG-strips (11 cm, pH 5-8,) obtained from BIO-RAD. We employed these IPG strips as they provided satisfactory resolution of the isolated DNA-binding proteins in the preliminary 2D PAGE experiments. Second dimension, SDS PAGE consisted of a 12% polyacrylamide resolving and 5% spacer gels, cast in standard BIO-RAD electrophoresis apparatus. Purified DNA-binding protein was dissolved in IEF sample buffer [40 mM Tris, pH: 10, 7 M urea, 2 M thiourea, and 1% C7BzO {3-(4-Heptyl)-phenyl-3-hydroxy-propyl-dimethylammonio- propane sulfonate}] and estimated spectrophotometrically after Bradford reaction. Each IPG strip was re-hydrated with rehydration buffer (8 M urea, 2% CHAPS, 50 mM DTT and 0.2% carrier ampholytes pH 3-10; BIO-RAD) pre-mixed with 200 µg of DNA-binding protein. IEF was carried out in a Protean IEF cell (BIO-RAD) with end voltage of 10,000, at 20° C for a total of 60,000 Vh. After IEF, IPG strips were stored at -80° C till their use in 2^{nd} dimension. First dimension electrophoresed IPG strips were incubated at room temperature for 15 min in 125 mM Tris-HCl, pH 6.8 containing 2.5 mM DTT and 2.3% (w/v) SDS, sealed on top of the spacer gel with 1% melted agarose in the same buffer. 2D PAGE gels were run at a constant current of 40 mA, till over-layered bromophenol-blue dye reached at the bottom of the gels. Both SDS and 2D PAGE gels were routinely stained with 0.2% coomassie brilliant blue R-250 in 5:1:4 of methanol: acetic acid: Milli-Q water over night and de-stained in the same solvent. 2D gel images were acquired using Fluor S Multiimager (BIO-RAD) and a visible light source. The image analysis was carried out using PDQuest image analysis software (BIO-RAD). All images were taken under uniform settings and 3-4 major spots in different parts of the gel were used for fixing the coordinates. Gels were also normalized for small variations in staining, using total optical density of the protein spots. Protein spots were allotted SSP (Standard Spot) numbers and protein spots from different gels were excised manually and pooled according to their SSP numbers.
**h. Sample preparation for MALDI-TOF analysis:** We performed gel runs in succession, pooled gel spots of each protein from all the gels and digested with trypsin. Sample preparation for MALDI TOF analysis was performed using standard protocols. Briefly, coomassie blue stained gel slices containing protein spots were washed in 50 mM ammonium bicarbonate and incubated briefly in the same buffer. Gel slices were washed further with 1:1 of 50 mM ammonium bicarbonate and 50% acetonitrile. Gel slices were dehydrated in acetonitrile and re-swollen in 20 mM ammonium bicarbonate, containing required amount of trypsin. In-gel digestions were performed with sequence grade bovine trypsin (Sigma) free of chymotryptic activity at a final concentration of 1:10 to 1:30 (trypsin : protein), at 37° C for overnight. Peptides were extracted twice with 5% Trifloroacetic acid (TFA) in 50% acetonitrile from in-gel digest, extracted peptides pooled, speed-vac concentrated and reconstituted in 8 µl of 0.1 % TFA in 50% acetonitrile and desalted using ZipTip C 18 columns (Millipore, USA). Purified peptides (in 0.1% TFA and 50% acetonitrile) were deposited on MALDI TOF plate, allowed to air dry, over-layered with α-cyano-4-hydroxy cinnamic acid (10 mg/ml, prepared in the same medium) matrix, air dried and used for MALDI TOF analysis.
**i. MALDI TOF study and analysis of the spectra:** We employed Voyager model: DE STR MALDI TOF Mass Spectrometer (PerSeptive Biosystems, Framingham, MA, USA) and MALDI mass spectra were recorded in the reflectance mode using delayed extraction and each measurement was performed using the following parameters: 20 kV acceleration voltage, 72% grid voltage, 175-220 ns delay time and low mass gate of 750. Spectra were accumulated from 100 laser shots. Peptide mass calibration was performed with external mass standards (Calmix 1 and 2; Applied Biosystems). MALDI TOF spectra were subjected to baseline correction, noise removal and peak detection employing standard procedures. From the data we eliminated peptide masses relating to trypsin, keratin and calibration fixture for arriving at the final peptide mass list. For most of the proteins, we recovered about 10-15 peptides on an average for each 10-kDa portion of the protein.
**j. PMF search analyses:** Snake protein databases are not available as yet. Therefore we expected PMF search of snake oocyte proteins should lead to identification of homologous proteins present in other databases. Thus we initiated PMF search in "All Taxonomy" mode employing 1-2 missed cleavages, "partial oxidation of methionine" and 200 ppm mass deviation. Perhaps due to non-availability of snake PMF database, for many proteins mass deviation had to be increased to 300-400 ppm for arriving at clear protein identities. However, in Mascot root mean square (RMS) ppm error (actual error utilized by mass data set for identifying the protein) variation was only 65-250 ppm. A marathon study conducted recently (Daniel et al, 2004) compared various PMF search parameters and suggested that a mass deviation of 400 ppm is necessary for arriving at meaningful protein identities, which confirms that mass deviation parameter employed in our PMF searches is very much in reasonable limits. Due to range and size of available NCBInr database and non-availability of snake protein database, we invariably employed NCBInr and used Mascot as well as profound search-tools for our PMF analyses. PMF searches were initiated employing 200 ppm mass deviation and increased to 300-400 ppm, wherever necessary for identifying proteins with significant protein scores. Based on the protein mass value and pI, we employed 1-2 missed cleavages and allowed 1 missed cleavage for each 10 kDa, mass of protein. More basic proteins shall possess higher content of basic amino acids- arginine / lysine, next to which trypsin cleaves the polypeptides and limitations in proteolysis shall require greater missed cleavage allowance for such proteins.
   Mass and pI range allowed in the search parameters certainly influences Estd Z score of proteins identified by Profound. Proteins with identical function in different taxonomy shall be homologous, but can vary considerably in their mass and pI values frequently. We expected that non-availability of snake protein database, requiring identification in terms of homologous proteins in other databases could possibly affect our PMF search results and provide only lower protein / Estd Z scores. We allowed 1unit of pI and 5 kDa of mass on either side of the experimentally observed protein pI and mass in all the PMF searches. We always looked for protein identities with closest mass and pI to the experimentally observed values of the proteins. In several cases we arrived at protein identities with sufficiently close mass and pI values to the snake oocyte protein, being investigated. However, in a few cases there were deviations, which appeared necessary for arriving at unique identities with best protein / Estd Z scores, as they alone were the best fits, identified both by Mascot as well as Profound. In some cases Profound identified a protein different than that identified by Mascot with acceptable score, while it also identified Mascot identified protein with good protein score. In such cases we considered and reported all the proteins identified with acceptable scores by these analyses. In many cases we initially employed Mascot and arrived at the protein mass, pI and organism at a reasonably good protein score (50 / 76 to 65 / 76) and employed this information in Profound, for identifying proteins with clearly acceptable Estd Z scores, which was shown in the data. PMF search of proteins employing *E. coli* instead of 'All Taxonomy', yielded identical protein / Estd Z scores for those actually identified as *E. coli* proteins by search in 'All Taxonomy' (see results), while others yielded only very insignificant proteins scores. This testified that proteins recovered by us did not originate from *E. coli*, particularly because *E. coli* genome is fully sequenced and all *E. coli* proteins are available in the database.
**k. Electro Spray Ionization Mass Spectrometry (ESI MS):** Few proteins, upon digestion yielded peptides sufficient for performing ESI MS analysis. Thus, we performed ESI MS of such protein digests for arriving at their internal sequence tags. MS/MS fragmentation spectra were obtained using a QSTAR Pulsar (ESI-Q-TOF) from PE Sciex (Toronto, Canada) with a nano spray source. TOF MS was obtained at 1000 V spraying voltage. Multiply charged species were subjected to MS/MS with collision energy ranging from 30-50 eV.
**l. NCBI BLAST analyses:** ESI MS study yielded us only 10-15 AA sequence tags of proteins. We performed BLAST of the sequence tags with "Search for Short, Nearly Exact Matches" tool of NCBI protein BLAST. As ESI MS cannot discriminate between AA "L & I" and "K & Q", we had to perform these BLAST searches in all possible combinations of these amino acids. Among the BLAST identified proteins with highest scores, we looked for proteins concurrently identified earlier by Mascot and Profound PMF searches and reported the results.

### Result:

In this study, total RNA was isolated from snake oocytes, mRNA purified and converted to cDNA as described in 'Methods'. Isolated mRNA was intact and cDNA synthesis gave transcripts between 0.5-4.5 kb, which are expected to produce proteins of a big range in size. Thus our library construction procedures are sound and library should express proteins of a wide range of molecular weight. Earlier, overexpression of proteins in GJ1158 bacteria has been shown to plateau by 3 hrs of induction (Bhandari and Gowrishankar, 1997). In view of this, snake oocyte cDNA library was induced for 3 3 hrs after growing to various levels between 0.2-0.8 OD units at 600 nm and total proteins resolved on 12.5% SDS PAGE. Protein expression increased with culture OD and reached its plateau by about 0.6 OD and excellent overexpression is seen between 0.6-0.8 OD upon induction for 3 hrs. Therefore these conditions were employed in all further experiments for induction of the library. Protein profiles of un-induced and induced control GJ1158 bacteria devoid of phagemids and un-induced library with the phagemids were similar, while protein profile of induced library showed significant changes as compared to the un-induced controls.

Proteins from un-induced and induced snake oocyte cDNA library were fractionated into soluble and insoluble fractions and analyzed on SDS PAGE. Greater proportion of library-expressed protein is present in the soluble fractions, which is in agreement with the fact that most of the GJ1158 expressed proteins are found in the soluble form (Bhandari and Gowrishankar, 1997). We dissolved insoluble portion of induced library protein in denaturing solvents and refolded the proteins as described in 'Methods'. We repeated the protein extraction and refolding step for the residual insoluble protein and finally recovered most of the expressed proteins in the soluble fraction. Thus our protein extraction and refolding procedure is successful in recovering most of the library-expressed proteins finally in the soluble form almost quantitatively. 2D PAGE profiles of "Un induced" and "Induced" proteins from snake oocyte cDNA library are shown in Fig. 1. Protein profile of the "Induced" shows several new protein spots (shown in purple circles), which are not present in the "Un-induced" control. Protein profile of the "Induced" clearly shows that total-snake oocyte cDNA-library expressed several proteins from the cloned cDNAs upon induction. Thus methods employed herein for library construction, expression of total cDNA library and extraction of proteins has been successful.

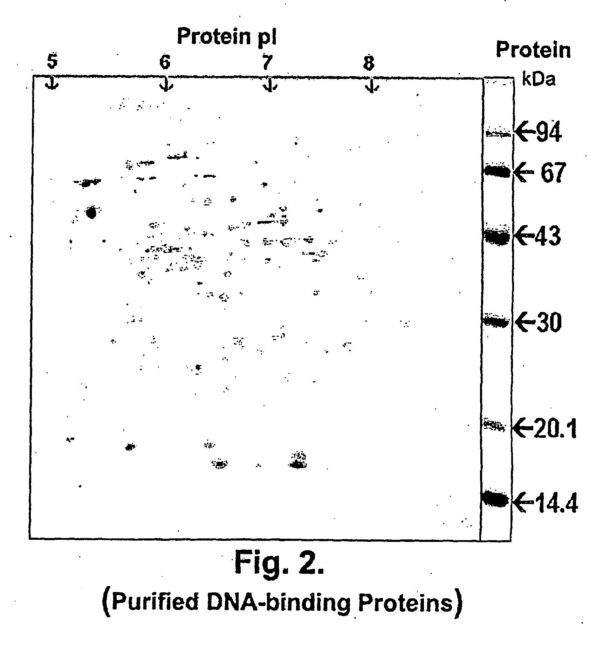

We isolated DNA-binding proteins from total library expressed protein, employing snake DNA-Sepharose column as described in 'Methods'. When un-induced library or control GJ1158 bacterial proteins were subjected to chromatography through DNA-Sepharose column under identical conditions, we hardly recovered any bound protein. In addition, purification of DNA-binding proteins in the presence or absence of added competitor *E. coli* DNA in the chromatography yielded nearly identical 2D PAGE pattern. This suggested that conditions employed in the chromatography permitted specific binding of snake DNA-binding proteins to snake DNA-Sepharose. During extraction and solubilization of library expressed protein, bacterial DNA fragments also get extracted into the soluble fraction. These fragments of *E. coli* DNA perhaps compete decisively with snake DNA-Sepharose for binding to *E. coli* DNA-binding proteins. This might explain the observed specificity of interaction between snake DNA-binding proteins and DNA-Sepharose. We processed the total protein recovered from 90 litters of induced culture of the library through snake DNA-Sepharose column in several batches and recovered ∼1.8 mg of DNA-binding protein expressed in the library. We expect a recovery of about 5 mg of overexpressed protein per liter culture in GJ1158 bacteria. Thus 90 liters of culture should yield ∼450 mg of overexpressed protein. As we isolate only the DNA-binding proteins from total library expressed protein, which forms only a very small proportion of total library expressed protein, low recovery of these proteins (∼0.4% of overexpressed protein) is perhaps justified. We resolved this protein on IEF followed by 2D PAGE and stained the gel with coomassie brilliant blue R-250. Profile of purified DNA-binding proteins showed in Fig. 2 reveals presence of several well-resolved spots ranging between ∼15-100 kDa in their mass values, and ∼5-9 in their pI. Thus methods employed herein for extraction and purification of DNA-binding proteins appears to be successful. Spots of resolved DNA-binding proteins were excised from 2D PAGE, subjected to in-gel digestion with trypsin and peptides recovered were used for MALDI TOF and ESI MS analyses.

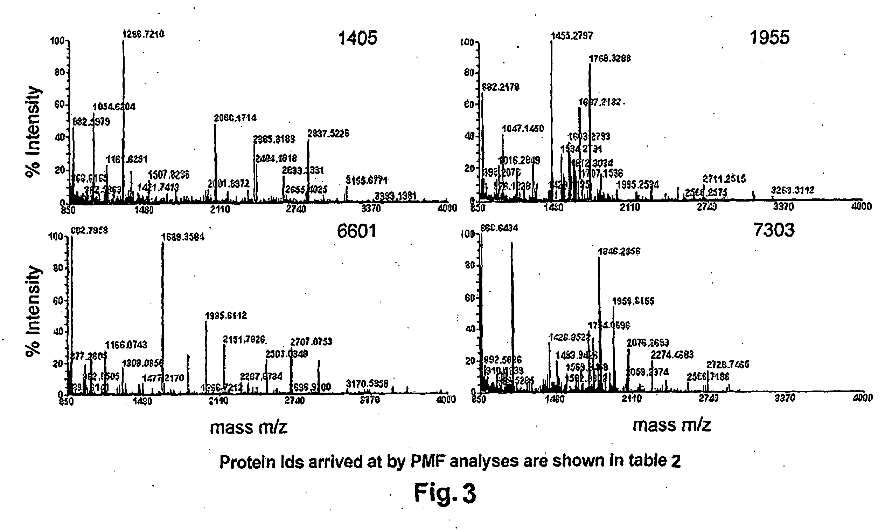

We collected spots of 80 DNA-binding proteins apparently possessing concentrations sufficient for MALDI TOF analysis and got reasonably good spectra for 37 proteins. Representative MALDI TOF spectra of four from these proteins are shown in Fig. 3. We recovered reasonably good PMF data for these proteins and succeeded in establishing the identity of all of them with reasonably good protein / Estd Z scores. Table 2 presents the data of 23 isolated snake oocyte DNA-binding proteins and their identities arrived at by these analyses. However, the remaining proteins identified are non DNA-binding, but are of bacterial origin, for which we offer a most plausible explanation (see later). When we subjected, each of the identified snake DNA-binding proteins to PMF analysis in "*E. coli*." instead of "All Taxonomy", with the parameters arrived at by the above procedure (Table 2) each analysis gave protein identities only with very low scores, clearly suggesting that these are not *E. coli* proteins. This is not expected if these were *E. coli* proteins, because *E. coli* genome is fully sequenced, database is available and *E. coli* proteins can be identified by PMF analyses without any problem.

In some cases we arrived at clear protein identity at 200 ppm mass error, either with Mascot / Profound or both. In other cases protein identity could be established only after employing 300-400 ppm mass errors. In many cases we established protein identity with significant scores either by Mascot or Profound, while the other identified the protein only with a score, lower than the significant score level, which was marked "(*)", next to the score values in Table 2. In a few cases, protein and / or Estd Z scores of identified proteins were slightly lower than the "significant score" level. We accepted these as Mascot and Profound independently or concurrently arrived at the same identity with reasonably good protein / Estd Z scores. In many cases, it was not possible to arrive at identity of proteins with significant scores in one go with Profound. In such cases we first arrived at reasonably good protein score by Mascot and employed mass and pI and / taxonomy of identified protein in Profound for arriving at clear identity (see Methods). In such cases the identity was appropriately marked and comment made at the bottom in Table 2. This strategy enabled us to arrive at clear identify of several snake oocyte proteins. Earlier, it was reported that it is relatively difficult to arrive at clear protein identity by Profound PMF search and thus it is thought to lead for a more accurate prediction of protein identity (Daniel et al., 2004). However, our experiences reveal that arriving at clear protein identity by Mascot is more difficult, thus leaving an impression that perhaps Mascot provides better accurate protein prediction. Non-availability of snake protein database might also be responsible for our difficulty in PMF searches and arriving at this view.

We subjected PMF analysis identified proteins to "Conserved Domain Database" search of NCBI for understanding their probable physiological function. We arrived at, the most likely physiological function of identified proteins, which are shown in Tables 3. It is interesting to note that most of the identified proteins (Table 3) appear to require DNA-binding property for their proposed physiological function, suggesting that these are perhaps true / functional DNA-binding proteins. Interestingly identified proteins belonging to a variety of organisms like microbes, pests, plants, flies and mammals are present in the list. Identification of proteins of different organism in snake oocytes does not necessarily mean that snake oocytes actually possesses these proteins, but only imply that isolated snake oocyte proteins are homologous with those reported earlier from such organism. DNA-binding proteins identified in this study include factors involved in transcription / signal transduction mechanisms, DNA-replication / recombination and repair, cell division and chromosome segregation / partitioning, pre-mRNA processing, nucleases and DNA-binding proteins.

In spite of employing rigorous chromatographic conditions few proteins (data not shown), although apparently devoid of DNA-binding property, got retained on DNA-Sepharose column and made their way into the group of DNA-binding proteins. We expressed the entire library of proteins, dissolved most of the library-expressed proteins and subjected them to chromatography through DNA-Sepharose column under native conditions. In cells under physiological conditions, various functions of cell and proteins required for these functions are strictly compartmentalized and thus biological systems do not permit non-functional protein-protein associations. During DNA-Sepharose affinity chromatography all native proteins coexisted together in solution. This could lead to interaction of some of the non-DNA-binding proteins with DNA-binding proteins through their motifs that promote protein-protein interaction. Beta lactamase is one of such protein originating from PT7T3D cloning vector, which is expressed in GJ1158 bacteria for hydrolyzing ampicillin, used as a selection marker for the bacteria. DNA-binding proteins also possess protein-protein interacting motifs (in addition to DNA-binding domains), form multi-protein complexes with other functional proteins containing similar motifs and associate with their target genes in the cells for performing their function. Therefore, DNA-binding proteins possess the ability to interact with other proteins and in a non-physiological system, like the affinity chromatography herein, proteins devoid of DNA-binding property could very well associate and make their way into the group of DNA-binding proteins. Further, such protein-protein (hydrophobic) interactions get stabilized during the washing and elution steps of the chromatography due to increased salt concentrations of the buffers. Recovery of proteins involved in protein-protein / protein-amino acid interactions and of periplasmic (usually hydrophobic) localization in the present study strengthens this argument and offers the most plausible reason for retrieving proteins devoid of DNA-binding property in our DNA-binding protein purification.

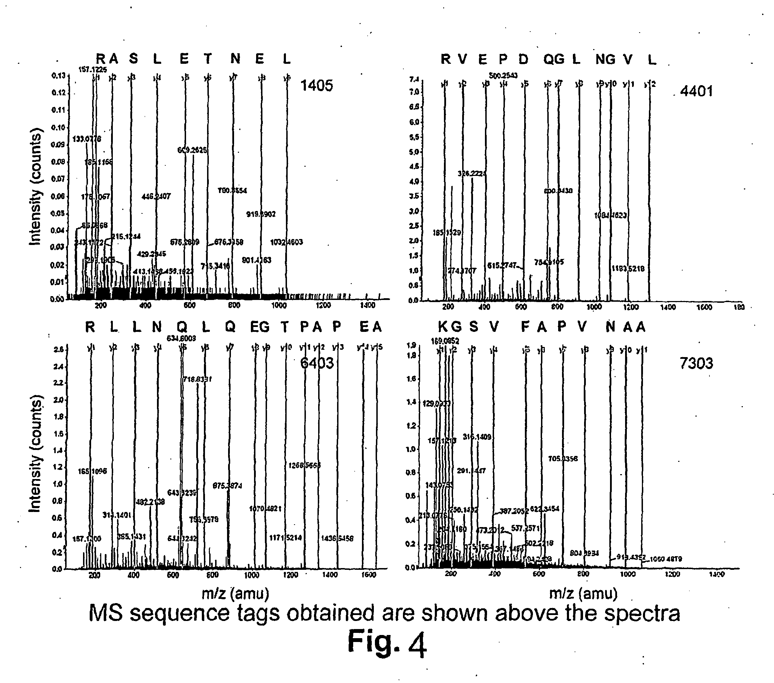

We performed ESI MS study, wherever peptide concentrations were sufficient, and recovered sequence tags of six proteins. Representative ESI MS spectra of four of the studied proteins are shown in Fig. 4. We performed database search of the sequence tags by NCBI BLAST analyses as described in "Methods". Interestingly, protein identities arrived at by PMF analysis for 3601, 4401, 6403 and 7305 were present among NCBI BLAST identified proteins with the highest scores. Protein identities obtained for 1405 and 7303 by PMF analyses were not present among the respective proteins identified by NCBI BLAST. It can be seen from Fig. 3 that MALDI TOF spectra obtained for proteins 1405 and 7303 are of good quality. Further PMF analyses yielded unique identities between Mascot and Profound searches and with good score at least by Profound (Table 2). Since proteins identified for 1405 and 7303 by PMF analysis are DNA-binding (Table 2) and as we recovered these proteins employing DNA-Sepharose chromatography, we conclude that identities of these proteins by PMF analyses are more reliable. Further, unlike NCBI BLAST, PMF analysis being based on information derived from a full-length protein is more reliable and supports our above conclusion. Sequence-tags arrived at for ESI MS studied proteins, protein identities from PMF and NCBI BLAST analyses, mass values of identified proteins and corresponding experimental values from 2D PAGE are shown in Table 4.

**Table 4**

| **Comparison of the proteins identities obtained by PMF analysis with those identified by NCBI BLAST, employing their ESI MS sequence tags:** | | | | |
|---|---|---|---|---|
| **Sl. NO.** | **SSP No.** | **Mass Tag of ESI MS sequenced peptide; Protein Mass and pI From 2D PAGE** | **Name of the protein, Protein mass, pI, Organism, Gene Identity and Match score, which were Identified by** | |
| | | | **PMF analysis** (Ma: Mascot & Pf: Profound) | **NCBI BLAST analysis** (EV: E. Value) |
| 1. | 1405 | LENTELSAR 22.70 and 5.30 | Another partner for ARF 1; 52.19; 5.80; [*Mus musculus*]; gi\|18204847 Ma: 70/77(*); Pf: 2.43 | HSP, HsIVU, Proteasome-related pepti-dase subunit; 19.093; 5.96; [*Shigella flexneri* 2a *str.301*]; gi\|24115226; EV: 29.00 |
| 2. | 3601 | VILVGNLGQD 31.00 and 6.00 | Single-stranded DNA-binding protein; 18.79; 5.40; [*Serratia marcescens*]; gi\|47270; Ma: 55 / 77(*): Pf: 2.43 | Single-stranded DNA-Binding protein; 18.809; 5.44; [*Serratia marcescens*] gi\|47270; EV: 0.71 |
| 3. | 4401 | LVGNLGQDPEVR 22.70 and 6.20 | ssDNA-binding protein; 18.963; 5.5; [*Shigella flexneri 2a str. 301*]; gi\|56384074 Ma: 28 / 57(*); Pf: 1.37(*) | ssDNA-binding protein; 18.809; 5.44; [*Shigella flexneri 2a str. 301*] gi\|56384074; EV: 0.006 |
| 4. | 6403 | AEPAPTGEQLQNLLR 21.40 and 7.50 | Hypothetical protein S1573; 19.99; 6.80; [*Shigella flexneri* 2a str. 24571]; gi\|30041221; Ma: 56 / 76(*); Pf: 2.43 | Hypothetical protein SF1458; 19.35; 8.40; [*Shigella flexneri 2a str. 301*] gi\|24112838; Sequence same as gi\|30041221; EV. 1e-04 |
| 5. | 7303 | AANVPAFVSGK 16.60 and > 8.00 | ATPases involved in chromosome partition-ing; 20.55; 9.30; [*Crocosphaera watsonii WH 8501*]; Ma: 54 / 77(*); Pf: 1.76 | DNA-binding protein HU-alpha (HU-2); 9.535; 9.57; [*Shigella flexneri 2a str. 301*] gi\|24115284; gi\|45525746; EV: 0.22 |
| 6. | 7305 | NTLENEFQG 18.80 and > 8.00 | Histone-like protein, located in outer membrane or nucleoid; 17.67; 9.70; [*Shigella flexneri 2a str. 301*]; gi\|24050382; Ma: 79 / 76; Pf: 2.43 | HLP, Histone-like protein; [*Shigella flexneri 2a str.301*]; Sequence same as gi\|24050382; 17.618; 9.69; gi\|24111613; EV: 39 |

| | | | | |
|---|---|---|---|---|
| 1. Protein score marked (*), imply that the score is lower than the required significant score level. Reason for accepting such 'protein identities', is explained in the text | | | | |

We started with a scarce tissue; isolated low-abundant proteins from total cDNA library expressed protein and characterized them employing the proteomics technology. Although, limitations in the amount of protein and non-availability of database caused some difficulties, we made an earnest attempt to overcome these difficulties employing novel methods, which are apparently useful for many working in similar area under such limitations. This entire exercise of constructing snake oocyte cDNA library, expression of total library, purification / identification of proteins employing the proteomics technology clearly demonstrates that this methodology is a viable proposition and possesses very potential applications. Our success in the isolation and identification of rear proteins expressed in a total tissue cDNA library provides a strong basis for developing a "Tissue Proteome Library', which is a very novel concept.

**Advantages of the present approach over the existing technologies**: At present overexpression of a protein is a long and stressful procedure. Respective cDNA of a protein is PCR amplified from a pool of tissue cDNAs, ligated into the correct reading frame of an expression vector, plasmids transformed into appropriate host cells and induced for expression of the protein. Methods are not prescribed in the literature that allows one to express a pool of cDNAs to overproduce their respective pool of proteins, which saves the effort and time in overexpression of all the transcripts individually. Therefore, the rationale and examples 1-3, provided as proof of concept provided herein is truly novel. This method allows one to achieve overexpression of a pool of cloned cDNAs into a pool of their respective proteins. Some of the extremely important classes of molecules like DNA-binding proteins are present at very low amounts in cells and it is extremely difficult even to identify them and their presence in cells and tissues. Isolation of such molecules from cells and tissues is extremely difficult and some times impossible because of their low abundance in and availability of such tissues. Some of the cells / tissues like tissue specimen samples from patients, oocytes and early embryos are available only in very limited or minute quantities. Therefore the method presented here allows for overexpression of a large number of proteins, for which mRNAs / cDNAs are present in their tissue / library. In addition, it allows us to identify and / isolate such proteins from the pool of proteins overexpressed in the tissue proteome library. This also stands as an important novelty of the present approach.

Researchers have been trying to synthesize combinatorial libraries of peptides, to look for specific ones that possess biological activities / therapeutic applications. The present approach provides an opportunity for expressing large number of peptides translated in incorrect reading frames and truncated. In an effort to combat diseases, biological systems very likely might express some of their proteins in incorrect frames to arrive at biologically active and therapeutically useful peptides. Such peptides might serve as therapeutic agents to control biological processes. There are no methods available at present to express / synthesize / overproduce biologically important / therapeutic peptides. Protein expression in incorrect frames leads to termination of translation (due to stop codons) and result in producing a large pool of biological peptides (see later) that might possess important biological properties / therapeutic applications. Thus our present approach stands as a novel method and source for generating the starting material for conducting studies for arriving at a pool of such important peptides. Several advanced methods in library construction are available. Transcripts present in as few as 10 cells or tissue specimen samples can be analyzed by protein expression and proteomics analysis employing PCR based amplification of cDNA pool and construction of expression libraries. Methods of library construction including normalization, referred to as subtraction libraries are also available, which increase the frequency of low abundant transcripts. Construction of subtraction libraries combined with cloning and induction of total cDNA library enables overexpression of even low abundant transcripts and their analysis / identification by the proteomics approach.

It is possible to develop plasmid or phagemid vectors specifically designed for cloning and overexpression of the total tissue cDNA in all the frames of an expression vector. The sequence elements needed in such vectors are specific 6-8 base specific restriction sites (*Rsa 1, Sfi 1, Sgf 1, Pme 1, Not 1* etc.) on the 5' and 3' termini of the cDNAs to be cloned, antibiotic resistance genes, specific amino acid tags (His-tag or GST-fusion tag etc.) on the 5' termini, that help in purification of all the expressed proteins, and general (T7 and T3) primer sequences on either side of the cDNAs to be cloned, required for amplification of the cloned cDNAs etc. BD Biosciences markets SMART cDNA library construction kits, which employs a phage expression vector. This vector is claimed to expresses proteins in all three frames, although each cloned cDNA integrated into the phage must be released as a phagemid, before they can be expressed. This needs a lot of standardized for each of the clones and thus they are not useful for expression of all the cloned cDNAs in one go. Therefore there is need for developing specific vectors that can express all the cloned cDNAs in one go, which are useful for developing tissue proteome libraries.

Since proteomics has already acquired automatic, high-throughput capabilities, it can advantageously be employed in analyzing and identifying proteins present even in mixtures containing large number of proteins. Above, total library expression approach can be combined with proteomics for analyzing the transcripts present even in clinical tissue specimens, oocytes and early embryos, which are available only in minute quantities. This facilitates comparison of protein profiles of such specimen samples with those of appropriate controls to assess changes in the protein patterns on one side and identifying unknown proteins present in them on the other. Important present day research problems of biology like nuclear reprogramming, embryonic differentiation, development, aging and disease can be resolved much faster by employing these methodologies which certainly enhance the pace of studies in proteomics, bio-physical, bio-chemical and molecular biology areas of research.

### REFERENCES

1. Amatschek, S., Koenig, U., Auer H., Steinlein, P., Pacher M., Gruenfelder, A., Dekan, G., Vogl, S., Kubista, E., Heider, K.H., Stratowa, C., Schreiber, M., Sommergruber, W. (2004) Tissue-wide expression profiling using cDNA subtraction and microarrays to identify tumor-specific genes. Cancer Res. 64, 844-856
2. Anderson, M., Blowers, D., Hewitt, N. Hegde, P., Breeze, A., Hampton, I. and Taylor, I. (1999), Refolding, purification and characterization of a loop deletion mutant of human Bcl-2 from bacterial inclusion bodies, Protein Expression and Purification 15, 162-170
3. Bhandari, P. and Gowrishankar, J. An Escherichia coli host strain useful for efficient overproduction of cloned gene products with NaCl as the inducer. (1997) J. Bacteriol. 179, 4403-4406
4. Campbell, K.H., Albersio, R., Lee, J. and Ritchie, W.A. (2001) Nuclear transfer in practice. Cloning Stem Cells, 3, 201-208
5. Campbell, K.H., McWhir, J., Ritchie, W.A. and Wilmut, I. (1996) Implications of cloning, Nature 380, 64-66
6. Chomczynski, P. and Sacchi, N. (1987) Single-step method of RNA isolation by acid-guanidinium thiocyanate-phenol-chloroform extraction; Anal. Biochem. 162,156-159
7. Coleman, A. (2002) Cloning 1, 185-200
8. Daniel, C.C., Gerhard, K., Kai, S., Helmut, E.M., Joachim, K. and Martin, B. (2004), Evaluation of algorithms for protein identification from sequence databases using mass spectrometry data, Proteomics 4, 619-628
9. Giltnane J. M. and Rimm D. L. (2004) Technology insight: Identification of biomarkers with tissue microarray technology. Nat. Clin. Pract. Oncol. 1,104-11
10. Hochstrasser, D.F., Sanchez, J.C. and Appel, R.D. (2002) Proteomics and its trends facing nature's complexity, Proteomics 2, 807-812
11. Joubert-Caron, R., Feuillard, J., Kohanna, S., Poirier, F., LeCaer, J.P., Schuhmacher, M., Bomkamm, G.W., Polack, A., Caron, M., Bladier, D. and Raphael, M.A. (1999) A computer-assisted two-dimensional gel electrophoresis approach for studying the variations in protein expression related to an induced functional repression of NFkappaB in lymphoblastoid cell lines. Electrophoresis 20, 1017-1026
12. Kadonaga, J.T. and Tjian, R. (1986) Affinity purification of sequence-specific DNA binding proteins: Proc. Natl. Acad. Sci. USA. 83, 5889-5893
13. Lieb, B., Carl, M., Hock, R., Gebaner, D. and Scheer, U. (1998) Identification of a novel mRNA-associated protein in oocytes of Pleurodeles waltl and Xenopus laevis, Exp. Cell Res. 245, 272-281
14. Laemmli, U.K. (1970) Cleavage of structural proteins during the assembly of the head of bacteriophage T4, Nature 227, 680-685
15. Mala, G., Paithankar, K. R., Jagannadham, M. V., Sundaram, C. S., Murthy, B. S.* and Singh, L (2007) Characterization of novel DNA-binding proteins expressed in snake oocyte cDNA library, Protein Expression and Purification 53, pp 164-178
16. Mann, M., Hendrickson, R.C. and Pandy, A. (2001) Analysis of proteins and proteomes by mass spectrometry. Annu. Rev. Biochem. 70, 437-473
17. Nordhoff, E., Egelhofer, V., Giavalisco, P., Eickhoff, H., Horn, M., Przewieslik, T., Theiss, D., Schneider, U., Lehrach, H. and Gobom, J. (2001) Large-gel two-dimensional electro-phoresis-matrix assisted laser desorption / ionization-time of flight-mass spectrometry: an analytical challenge for studying complex protein mixtures. Electrophoresis 22, 2844-2855
18. O'Farrell, P.H. (1975), High-resolution two-dimensional electrophoresis of proteins. J. Biol Chem. 250, 4007-4021
19. Panda A.K. (2003) Bioprocessing of therapeutic proteins from the inclusion bodies of E. coli. Adv. Biochem. Eng. Biotechnol. 85, 43-93
20. Paynton, B.V. (1998) RNA-binding proteins in mouse oocytes and embryos: expression of genes encoding Y box, DEAD box RNA helicase, and polyA binding proteins, Dev. Genet. 23, 285-298
21. Ravassard P., Icard-Liepkalns C., Mallet J., Dumas Milne Edwards J.B. (1997) cDNA libraries from a low amount of cells. Methods Mol Biol. 67, 317-29
22. Ryabova, I.V., Virtanen, I. and Coux, O.M. (1994) Distribution of prosome proteins and their relationship with the cytoskeleton in oogenesis of Xenopus laevis, Mol. Reprod. Dev. 37, 195-203
23. Sambrook, J., Fritsch, E.F., Maniatis, T. (1989) Cold Spring Harbor Laboratory Manual Vol: 1
24. Solter, D. (2000) Mammalian Cloning: advances and limitations, Nat. Rev. Genet. 1, 199-207
25. Tsunoda, Y. and Kato, Y. (2002) Recent progress and problems in animal cloning; Differentiation 69, 158-161
26. User manual (2001) The QIAexpressionist Protocols 10-p80 & 17-p90
27. Vaibhav, C.C., Subhashani, C., Dhople, V.M., Sundaram, C.S., Jagannadham, M.V., Kumar, K.N., Srinivas, P.N.B.S., Mythili, R., Rao, M.K., Kulkarni, M.K., Hegde, S., Hegde, A.S., Samual, C., Santosh, V., Singh, L. and Sirdeshmukh, R. (2005) Differential protein expression in human gliomas and molecular insights. Proteomics 5, 1167-1177
28. Westhusin, M.E., Long, C.R., Shin, T., Hill, J.R., Looney, C.R., Pryor, J.H. and Piedrahita, J.A. (2001) Cloning to reproduce desired genotypes; Theriogenology 55, 35-49
29. Wilmut, I., Schnieke, A.E., McWhir, J., Kind, A.J. and Campbell, K.H. (1997) Viable- offspring derived from fetal and adult mammalian cells. Nature 385, 810-813
30. Wulfkuhle, J.D., Liotta, L.A., Petricoin, E.F. (2003) Proteomic applications for the early detection of cancer, Nat. Rev. Cancer 3, 267-275

## Claims

1. A method to develop a tissue proteome library wherein the said method comprises the steps of:-
a. isolating the total RNA and mRNA from a tissue,
b. synthesizing total cDNA pool from the mRNA obtained in step a,
c. amplifying the recovered cDNA obtained from step b, using primers having specific restriction enzyme sites,
d. digesting the PCR amplified cDNA obtained in step c, with respective restriction enzymes,
e. cloning the digested cDNA obtained from step d, in all the 5'→3' directional frames of expression vectors,
f. transforming the plasmid pool obtained in step e, into bacteria by electroporation,
g. growing and inducing the bacterial culture obtained in step f, for protein expression,
h. isolating the expressed proteins in the bacteria obtained from step g, and
i. resolving isolated proteins, identifying individual proteins and expression levels of total proteins obtained from step h.

2. A method to develop a tissue proteome library as claimed in claim 1, wherein the restriction enzyme employed is a type II restriction enzyme consisting of 5 or more base restriction enzymes that generates cohesive termini upon cleavage.

3. A method to develop a tissue proteome library as claimed in claim 1, wherein the commercially available cDNA library or synthesized total cDNA library is directionally cloned into expression vectors in either one or all 3 possible frames.

4. A method to develop a tissue proteome library as claimed in claim 1, wherein the method comprises over expressing said proteins from step (g) to produce a total library of proteins from said tissue.

5. A method to develop a tissue proteome library as claimed in claim 1, wherein the pool of cDNA is derived from biomaterial belonging to the group consisting of eukaryotic cells, plant cells, insect cells and mammalian tissues, clinical tissue specimen samples, tissue culture grown cells, oocytes, zygotes, embryos and purified cellular organelles, tissue or whole organism.

6. A method to develop a tissue proteome library as claimed in claim 1, by cloning a pool of normalized population of cDNAs.

7. A method to develop a tissue proteome library as claimed in claim 1, wherein cloning / expression of a pool of cDNAs in expression vectors co-cloned and or co-expressed with either protease inhibitors or protein stabilizing agents.

8. A tissue proteome library as prepared by the method as claimed in claims 1-7, wherein it is useful in making a representative library of the expressed proteins and over expressing a large number of transcripts (mRNAs) present in a given tissue.

9. A tissue proteome library as claimed in claim 8, wherein the library is useful for various applications selected from the group consisting of overproduction, electrophoretic separation and proteomic analysis / identification of all the proteins from their mRNAs present in the tissue.

10. A tissue proteome library as claimed in claim 8, wherein the library is useful for identification of novel proteins even if truncated products of cDNAs are cloned and expressed or partially degraded proteins of > 10 kDa are produced during protein purification from bacteria.

11. A tissue proteome library as claimed in claim 8, wherein the library is useful for identification of novel proteins / transcripts present in the tissue.

12. The tissue proteome library as claimed in claim 8, wherein the library is useful for identification of low abundant proteins/ transcripts present in the tissue.

13. The tissue proteome library as claimed in claim 8, wherein cDNAs restriction digested with 5 or more nucleotide restriction enzymes from constructed or commercial or pre-made cDNA libraries.

14. Use of the tissue proteome library as claimed in claim 8, for various applications selected from the group consisting of, producing pools of biological molecules like mRNAs / cDNAs / proteins / peptides for research or other purposes like industrial, therapeutic, bio-medical, health-related, biotechnology, food technology and cosmetic purposes including studies and or applications thereof.

15. Use of the tissue proteome library as claimed in claim 8, for analyzing the total cellular protein expression of a tissue selected from the group consisting of expression and or isolation / identification of all or any sub-group of expressed proteins like membrane proteins, DNA-binding proteins, lipoproteins, nuclear proteins, transcription factors, signaling proteins, ribosomal proteins, mitochondrial proteins or any other group of proteins.

16. Use of the tissue proteome library as claimed in claim 8, for various applications selected from the group consisting of analysis of expressed protein by chromatography, 1D or 2D polyacrylamide gel electrophoresis (PAGE), Iso-electrophoresis (IEF), high performance liquid chromatography (HPLC), Matrix Assisted Laser Desorption-Ionization Time of Flight (MALDI TOF), Electro Spray Ionization Mass Spectrometry (ESI MS).

17. Use of the tissue proteome library as claimed in claim 8, for identification of expressed proteins in said host cell that possess a molecular weight of at least 10 kDa.

18. Use of the tissue proteome library as claimed in claim 8, for generation, identification, usage and application of a specific / pool of biological peptides from any biomaterials as claimed in claim 5.

## Patentansprüche

1. Verfahren zur Entwicklung einer Gewebe-Proteombibliothek, bei dem man die folgenden Schritte durchführt:
a. Isolieren der Gesamt-RNA und mRNA aus einem Gewebe,
b. Synthetisieren einer cDNA-Gesamtfraktion von der in Schritt a erhaltenen mRNA,
c. Amplifizieren der aus Schritt b erhaltenen gewonnenen cDNA unter Verwendung von Primern mit spezifischen Restriktionsenzymstellen,
d. Verdauen der in Schritt c erhaltenen PCR-amplifizierten cDNA mit entsprechenden Restriktionsenzymen,
e. Klonieren der aus Schritt d erhaltenen verdauten cDNA in allen Leserastern in 5'→3' - Richtung von Expressionsvektoren,
f. Transformieren der in Schritt e erhaltenen Plasmidfraktion in Bakterien mittels Elektroporation,
g. Kultivieren und Induzieren der in Schritt f erhaltenen Bakterienkultur zur Proteinexpression,
h. Isolieren der exprimierten Proteine in den aus Schritt g erhaltenen Bakterien und
i. Auftrennen isolierter Proteine, Identifizieren einzelner Proteine und Expressionsniveaus der aus Schritt h erhaltenen Gesamtproteine.

2. Verfahren zur Entwicklung einer Gewebe-Proteombibliothek nach Anspruch 1, wobei es sich bei dem eingesetzten Restriktionsenzym um ein Typ-II-Restriktionsenzym, bestehend aus mindestens 5-Basen-Restriktionsenzymen, handelt, wodurch bei der Spaltung kohäsive Enden erzeugt werden.

3. Verfahren zur Entwicklung einer Gewebe-Proteombibliothek nach Anspruch 1, wobei die kommerziell erhältliche cDNA-Bibliothek oder die synthetisierte cDNA-Gesamtbibliothek in Expressionsvektoren entweder in einem oder in allen der 3 möglichen Raster gerichtet kloniert wird.

4. Verfahren zur Entwicklung einer Gewebe-Proteombibliothek nach Anspruch 1, bei dem man die Proteine aus Schritt (g) unter Erhalt einer Gesamtbibliothek von Proteinen aus dem Gewebe überexprimiert.

5. Verfahren zur Entwicklung einer Gewebe-Proteombibliothek nach Anspruch 1, wobei die cDNA-Fraktion aus Biomaterial gewonnen wird, das zur Gruppe eukaryontische Zellen, Pflanzenzellen, Insektenzellen und Säugergewebe, klinische Gewebepräparateproben, in Zellkultur gewachsene Zellen, Oozyten, Zygoten, Embryos und aufgereinigte Zellorganellen, Gewebe oder ganzer Organismus gehört.

6. Verfahren zur Entwicklung einer Gewebe-Proteombibliothek nach Anspruch 1 durch Klonieren einer Fraktion einer normierten Population von cDNAs.

7. Verfahren zur Entwicklung einer Gewebe-Proteombibliothek nach Anspruch 1, wobei die Klonierung bzw. Expression einer Fraktion von cDNAs in Expressionsvektoren als Co-Klonierung bzw. Co-Expression entweder mit Protease-Inhibitoren oder Protein-Stabilisatoren.

8. Gewebe-Proteombibliothek, wie mit dem Verfahren nach Ansprüchen 1-7 hergestellt, wobei sie sich zur Herstellung einer repräsentativen Bibliothek der exprimierten Proteine und Überexpression einer großen Anzahl von in einem gegebenen Gewebe vorliegenden Transkripten (mRNAs) eignet.

9. Gewebe-Proteombibliothek nach Anspruch 8, wobei sich die Bibliothek für verschiedene Anwendungen, ausgewählt aus der Gruppe Überproduktion, elektrophoretische Trennung und Proteomanalyse bzw. Identifizierung aller Proteine von ihren im Gewebe vorliegenden mRNAs, eignet.

10. Gewebe-Proteombibliothek nach Anspruch 8, wobei sich die Bibliothek zur Identifizierung neuer Proteine eignet, selbst wenn verkürzte cDNA-Produkte kloniert und exprimiert oder teilweise abgebaute Proteine von >10 kDa während der Proteinaufreinigung aus Bakterien produziert werden.

11. Gewebe-Proteombibliothek nach Anspruch 8, wobei sich die Bibliothek zur Identifizierung im Gewebe vorliegender neuer Proteine bzw. Transkripte eignet.

12. Gewebe-Proteombibliothek nach Anspruch 8, wobei sich die Bibliothek zur Identifizierung im Gewebe vorliegender Proteine bzw. Transkripte mit geringer Häufigkeit eignet.

13. Gewebe-Proteombibliothek nach Anspruch 8, wobei cDNAs restriktionsverdaut mit 5 oder mehr Nukleotid-Restriktionsenzymen aus konstruierten oder kommerziellen oder vorgefertigten cDNA-Bibliotheken.

14. Verwendung der Gewebe-Proteombibliothek nach Anspruch 8 für verschiedene Anwendungen, ausgewählt aus der Gruppe Herstellen von Fraktionen biologischer Moleküle wie mRNAs / cDNAs / Proteine / Peptide zu Forschungs- und anderen Zwecken wie großtechnischen, therapeutischen, biomedizinischen, gesundheitsverwandten, biotechnologischen, lebensmitteltechnologischen und kosmetischen Zwecken, einschließlich Studien und/oder Anwendungen davon.

15. Verwendung der Gewebe-Proteombibliothek nach Anspruch 8 zur Analyse der gesamten zellulären Proteinexpression eines Gewebes, ausgewählt aus der Gruppe Expression und/oder Isolierung bzw. Identifizierung aller Untergruppen oder einer Untergruppe exprimierter Proteine wie Membranproteine, DNA bindender Proteine, Lipoproteine, zellkernproteine, Transkriptionsfaktoren, signalgebender Proteine, ribosomaler Proteine, mitochondrialer Proteine oder einer anderen Gruppe von Proteinen.

16. Verwendung der Gewebe-Proteombibliothek nach Anspruch 8 für verschiedene Anwendungen, ausgewählt aus der Gruppe Analyse exprimierten Proteins mittels Chromatographie, 1D- oder 2D-Polyacrylamid-Gelelektrophorese (PAGE), Isoelektrophorese (IEF), Hochleistungs-Flüssigkeitschromatographie (HPLC), Matrix Assisted Laser Desorption-Ionization Time of Flight [matrixgestützte Laser-Desorption-Ionisation-Flugzeit] (MALDI-TOF), Elektrospray-Ionisation-Massenspektrometrie (ESI-MS).

17. Verwendung der Gewebe-Proteombibliothek nach Anspruch 8 zur Identifizierung exprimierter Proteine in der Wirtszelle, die ein Molekulargewicht von mindestens 10 kDa besitzen.

18. Verwendung der Gewebe-Proteombibliothek nach Anspruch 8 zur Erzeugung, Identifizierung, Verwendung und Anwendung eines spezifischen biologischen Peptids bzw. einer Fraktion biologischer Peptide aus beliebigen Biomaterialien nach Anspruch 5.

## Revendications

1. Procédé pour développer une banque protéomique tissulaire, ledit procédé comprenant les étapes consistant :
a. à isoler l'ARN total et l'ARNm provenant d'un tissu,
b. à synthétiser le pool d'ADNc total provenant de l'ARNm obtenu dans l'étape a,
c. à amplifier l'ADNc récupéré obtenu dans l'étape b, par utilisation d'amorces ayant des sites spécifiques d'enzymes de restriction,
d. à digérer l'ADN amplifié par PCR obtenu dans l'étape c à l'aide d'enzymes de restriction respectives,
e. à cloner l'ADNc digéré obtenu dans l'étape d dans tous les cadres directionnels 5'→3' de vecteurs d'expression,
f. à transformer le pool plasmidique obtenu dans l'étape e dans des bactéries par électroporation,
g. à cultiver et induire la culture bactérienne obtenue dans l'étape f pour une expression de protéines,
h. à isoler les protéines exprimées dans les bactéries obtenues dans l'étape g, et
i. à séparer les protéines isolées, en identifiant les protéines individuelles et les niveaux d'expression des protéines totales obtenues dans l'étape h.

2. Procédé pour développer une banque protéomique tissulaire selon la revendication 1, dans lequel l'enzyme de restriction utilisée est une enzyme de restriction de type II consistant en des enzymes de restriction à 5 bases ou plus, qui produisent des sites terminaux cohésifs après clivage.

3. Procédé pour développer une banque protéomique tissulaire selon la revendication 1, dans lequel une banque d'ADNc du commerce ou une banque d'ADNc totale synthétisée est soumise à un clonage directionnel dans des vecteurs d'expression, dans l'un des trois cadres possibles, ou dans tous.

4. Procédé pour développer une banque protéomique tissulaire selon la revendication 1, le procédé comprenant la surexpression desdites protéines provenant de l'étape (g) pour produire une banque totale de protéines provenant dudit tissu.

5. Procédé pour développer une banque protéomique tissulaire selon la revendication 1, dans lequel le pool d'ADNc est obtenu à partir d'un biomatériau appartenant au groupe consistant en les cellules eucaryotes, les cellules végétales, les cellules d'insectes et les tissus de mammifères, les échantillons de spécimens de tissu clinique, les cellules cultivées en culture tissulaire, les ovocytes, les zygotes, les embryons et les organelles cellulaires purifiés, un tissu, ou un organisme entier.

6. Procédé pour développer une banque protéomique tissulaire selon la revendication 1, par clonage d'un pool d'une population normalisée d'ADNc.

7. Procédé pour développer une banque protéomique tissulaire selon la revendication 1, dans lequel le clonage/l'expression d'un pool d'ADNc dans des vecteurs d'expression co-clonés et/ou co-exprimés avec des inhibiteurs de protéases ou des agents de stabilisation des protéines.

8. Banque protéomique tissulaire telle que préparée par le procédé selon les revendications 1-7, qui est utile pour fabriquer une banque représentative de vecteurs d'expression et surexprimer un grand nombre de produits de transcription (ARNm) présents dans un tissu donné.

9. Banque protéomique tissulaire selon la revendication 8, la banque étant utile pour différentes applications choisies dans le groupe consistant en une surproduction, une séparation électrophorétique et une analyse/identification protéomique de toutes les protéines provenant de leurs ARNm présents dans le tissu.

10. Banque protéomique tissulaire selon la revendication 8, la banque étant utile pour l'identification de nouvelles protéines, même si des produits tronqués d'ADNc sont clonés et exprimés, ou que des protéines partiellement dégradées, de plus de 10 kDa, soient produites pendant la purification des protéines provenant de bactéries.

11. Banque protéomique tissulaire selon la revendication 8, la banque étant utile pour identifier de nouvelles protéines/de nouveaux produits de transcription présents dans le tissu.

12. Banque protéomique tissulaire selon la revendication 8, la banque étant utile pour l'identification de protéines/produits de transcription peu abondants présents dans le tissu.

13. Banque protéomique tissulaire selon la revendication 8, dans laquelle des ADNc digérés par restriction à l'aide d'enzymes de restriction à 5 nucléotides ou plus, à partir de banques d'ADNc construites, ou du commerce, ou préfabriquées.

14. Utilisation de la banque protéomique tissulaire selon la revendication 8 pour différentes applications choisies dans le groupe consistant en la production de pools de molécules biologiques telles que des ARNm/des ADNc/des protéines/des peptides pour la recherche ou d'autres objectifs, tels que des objectifs industriels, thérapeutiques, biomédicaux, liés à la santé, biotechnologiques, liés à la technologie des produits alimentaires, et cosmétiques, y compris les études et/ou les applications correspondantes.

15. Utilisation de la banque protéomique tissulaire selon la revendication 8 pour analyser l'expression cellulaire totale de protéines d'un tissu, choisie dans le groupe consistant en l'expression et/ou l'isolement/l'identification de tous les sous-groupes ou de tout sous-groupe de protéines exprimées, telles que des protéines membranaires, des protéines de liaison à l'ADN, des lipoprotéines, des protéines nucléaires, des facteurs de transcription, des protéines de signalisation, des protéines ribosomales, des protéines mitochondriales ou tout autre groupe de protéines.

16. Utilisation de la banque protéomique tissulaire selon la revendication 8 pour différentes applications choisies dans le groupe consistant en l'analyse de la protéine exprimée par chromatographie, électrophorèse sur gel de polyacrylamide (PAGE) 1D ou 2D, isoélectrophorèse (IEF), chromatographie en phase liquide hautes performances (HPLC), la désorption-ionisation laser assistée par une matrice avec analyseur à temps de vol (MALDI TOF), la spectrométrie de masse par ionisation et électropulvérisation (ESI MS).

17. Utilisation de la banque protéomique tissulaire selon la revendication 8, pour identifier des protéines exprimées dans ladite cellule hôte, qui ont une masse moléculaire d'au moins à 10 kDa.

18. Utilisation de la banque protéomique tissulaire selon la revendication 8 pour la production, l'identification, l'utilisation et l'application d'un peptide biologique spécifique ou d'un pool de peptides biologiques provenant de tous biomatériaux tels que revendiqués dans la revendication 5.
